# EUROPEAN PATENT APPLICATION

(11) **EP 2 527 320 A1**
(43) Date of publication of application: **28.11.2012**
(21) Application number: 11167798.5
(22) Date of filing: 27.05.2011
(51) Int. Cl.: C07C 229/34, C07C 239/20, C07C 271/22, C07C 311/19, C07F 7/10, C07F 7/18

(54) **Preparation of Sitagliptin Intermediates**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The invention relates to the preparation of chiral compounds, in particular to the preparation of chiral compounds which may be used as intermediates for the preparation of anti-diabetic agents, preferably sitagliptin.

## Description

### Field of the Invention

The present invention relates to the preparation of chiral compounds, in particular to the preparation of chiral compounds which may be used as intermediates for the preparation of anti-diabetic agents, preferably sitagliptin.

### Background Prior Art

Type II diabetes mellitus (T2DM) is a global epidemic. Therefore, the research is oriented in the development of selective inhibitors of the enzyme DPP-IV as a promising new treatment for the type II diabetes.

Sitagliptin (CAS Registry Number 486460-32-6. IUPAC Name: (*R*)-4-oxo-4-[3-(trifluoromethyl)-5,6-dihydro[1,2,4]triazolo[4,3-a]pyrazin-7(8*H*)-yl]-1-(2,4,5-trifluorophenyl)butan-2-amin) is an anti-diabetic agent and a potent inhibitor of the DPP-IV. It is represented by the structure: 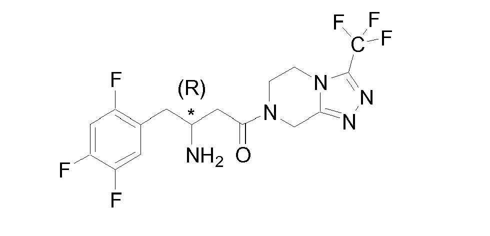 There is a constant search for improved synthetic protocols for key intermediates, in particular (β-amino acid intermediates of the formula I, 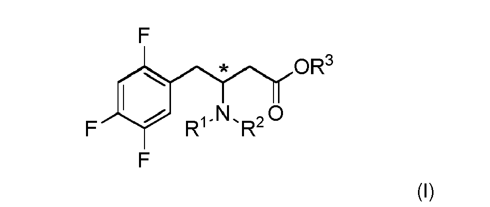 for the synthesis of sitagliptin.

WO 03/004498 disclose a method for producing the carboxylic acid of the (β-amino acid intermediate of the formula I, which is performed through a 2,3,5-trifluorobenzylbromide intermediate, where enantioselectivity was induced by the use of unusual dihydropyrazine chiral auxiliaries. In the last steps, diazomethane, which is an explosive reagent, and stoichoimetric ammounts of silver salts are included in the synthetic protocol which are very expensive and therefore unsuitable reagents for industrial synthesis.

Other synthetic approaches include asymmetric hydrogenation of β-enamino acid intermediates. The asymmetric hydrogenation reactions are conducted in the presence of expensive metal catalysts like rhodium in combination with chiral phosphine/diphosphine ligands (WO 03/004498, Kubryl, M.; et. al. Tetrahedron Asymmetry 2006, 17, 205). In some cases also expensive ruthenium metal catalysts are used (WO 09/064476, WO 04/085378, WO 05/097733, WO 06/081151, Hsiao, Y.; et. al. J. Am. Chem. Soc., 2004, 126, 9918). Hydrogenation with cheaper achiral catalysts involving a chiral derivatisation of enamines is also known (WO 04/085661 ).

Also known are synthetic strategies, which are based on the chemocatalytic selective reduction of β-keto esters in the presence of ruthenium or rhodium diphosphine chiral cataylsts (WO 04/087650, US 2009/0192326; US 2006/0052382; Hansen, K. B.; et. al. J. Am. Chem. Soc. 2009, 131, 8798.; Hansen K. B.; et. al. Org. Proc. Res. Dev. 2005, 9, 634-639).

WO 09/045507 discloses a biocatalytic approach to sitagliptin where an enantioselective step was performed using an appropriate enzymes (ketoreductase) for the asymmetric reduction of the β-carbonyl part of the molecule to form than the β-hydroxy intermediates of the formula IV. The transformation of the obtained chiral hydroxyl intermediates to the final sitagliptin precursors was performed via azetidinone intermediates. It is well known that this step is very difficult to establish. Disadvantages of these protocols are also: reactions at high pressures (250 psi), the use of very expensive metal chiral catalysts (Rh or Ru), low stereoselectivity and product contamination with rhodium and consequently hard purification protocols of final compound.

It has been also shown that rhodium or ruthenium asymmetric catalytic hydrogenation of β-keto esters through enamines can be replaced by the an efficient biocatalytic process using special enzymes transaminases, which improve the efficiency of sitagliptin manufacturing up to 99.95% enantiomeric excess (Savile, C. K.; et. al. Science 2010, 329, 305 and references cited therein). This enzymatic route features direct amination of the prochiral sitagliptin ketone to provide the enantiopure sitagliptin, followed by phosphate salt formation to provide the final sitagliptin phosphate. It is well known that enzymatic reactions offer an environmentally friendly approach to the synthesis of final molecules but on the other hand the availability and especially price of special enzymes (isolation protocols etc.) represent a inconsiderable disadvantage of a biocatalytic process. WO 09/045507 discloses protocols for the synthesis of a β-hydroxy intermediate and the β-amino acid intermediate of formula I.

There is also disclosed an intermediate of the formula II 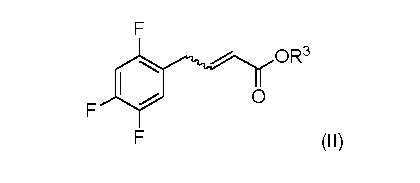 with R³ being methyl, but no experimental procedure, no evidence and any other signs are devoted to this intermediate (WO 2010/122578). All synthetic strategies disclosed (WO 2010/122578) are experimentally complicated, involve relatively many synthetic steps and some of them are conducted under extreme reaction conditions (temperature up to -50 °C; dry conditions etc.). The efficiency and especially the selectivity of some individual synthetic steps are modest and consequently influence the lower overall yields of the process.

Liu et al. discloses an asymmetric synthesis of sitagliptin over 9-10 steps, with the overall 31% yield and 99.5% enantiomeric excess (Liu, F.; et. al. J. Chem. Res. 4, 2010, 230-232). The synthetic strategy involving also an intermediate of formula (II) presents an efficient and high selective approach to sitagliptin but on the other hand offers also a lot of disadvantages. One of these disadvantages is the long and complicated 5 steps process to obtain the intermediate of the formula II.

Some other important disadvantages are: some steps are conducted under extreme conditions (-78 °C) where special equipment is also needed; the use of extremely hazardous reagents like BuLi strongly needed for performing the aza-Michael reaction under these conditions; some steps include CH₂Cl₂ as an volatile, toxic and especially non-industrial and non-environmentally friendly reaction medium;

Therefore, it was an object of the present invention to provide an improved, simple, cost-beneficial, industrial friendlier and environmentally friendly process for the preparation of an intermediate of formula I.

It was another object of the present invention to provide an improved process for the preparation of an intermediate of formula I starting from the intermediate of the formula II.

It was yet another object of the present invention to provide new intermediates suitable for the preparation of anti-diabetic agents, preferably sitagliptin.

### Summary of the Invention

The present invention relates to a process for the preparation of an intermediate of formula I 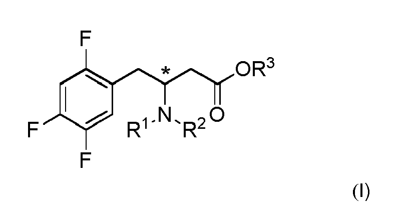 wherein R¹ and R² are identical or different, and are independently selected from
(i) hydrogen;
(ii) optionally chiral alkyl residues, having from 1 to 12 carbon atoms, wherein the alkyl residues are optionally aryl and/or aryloxy substituted;
(iii) optionally chiral alkyloxy residues, having from 1 to 12 carbon atoms, wherein the alkyloxy residues are optionally aryl substituted;
(iv) optionally chiral aryl residues, having from 6 to 24 carbon atoms, wherein the aryl residues are optionally alkyl and/or alkyloxy substituted;
(v) optionally chiral aryloxy residues, having from 6 to 24 carbon atoms, wherein the aryloxy residues are optionally alkyl substituted;
(vi) benzyl;
(vii) optionally chiral alkaloyl residues, having from 2 to 13 carbon atoms, wherein the alkaloyl residues are optionally aryl substituted;
(viii) optionally chiral aroyl residues, having from 7 to 25 carbon atoms, wherein the aryloxy residues are optionally alkyl substituted;
(ix) optionally chiral alkoxycarbonyl residues, having from 2 to 13 carbon atoms;
(x) optionally chiral aryloxycarbonyl residues, having from 7 to 25 carbon atoms;
(xi) tosyl;
(xii) optionally chiral silyl residues, having from 3 to 15 carbon atoms; and
(xiii) optionally chiral silyloxy residues, having from 3 to 15 carbon atoms;
   wherein R³ is selected from alkyl residues having from 1 to 6 carbon atoms;
   the process comprising the steps of:
   (a) providing an intermediate of formula II, 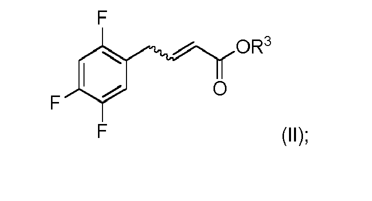
   (b) reacting the intermediate of formula II with an amine of formula III

      HNR¹R² (III)

      wherein R¹ and R² are as defined above, in a protic solvent, particularly in water; or in a mixture of protic solvents, wherein the mixture particularly comprises water; or without adding of solvents in step (b); to obtain an intermediate of formula I.

The present invention represents an improvement over the known methodologies. The developed reactions are conducted under mild reaction conditions; simple, non-hazardous and the commercially available reagents may be used; reactions are performed under environmentally friendly reaction conditions using water as a "green" solvent or using no solvent at all; there is no need for special equipment for efficiency of the process; and much less reaction steps considering previous patents and literature are necessary to obtain an intermediate of formula I.

Other aspects and further preferred embodiments are set out as defined in the claims and in the detailed description of the invention.

### Definitions

The term "intermediate" as used herein shall be understood as including compounds which are isolated from a reaction mixture and compounds which are not isolated from a reaction mixture.

The term "room temperature" or "ambient temperature" used herein will be understood by the person skilled in the art as referring to a temperature between about 20 °C and about 30 °C, particularly between 20 °C and 30 °C.

The term "without adding of solvents" used herein is meant to refer to conditions in which no solvent or solvents are added additionally to the reactants, particularly in which no solvent or solvents are added additionally to any adducts, and if present, any catalysts, any ligands, any bases, any acids, any organocatalysts, any promoters, and any surfactants. Thus, the step (b) of the process as defined below is essentially carried out in the absence of a solvent or solvents. In the event that one or more reactant, particularly an adduct, a catalyst, a ligand, a base, an acid, an organocatalyst, a promoter, or a surfactant, could be considered as solvent, the term "without adding of solvents" used herein is meant to refer to conditions in which no solvent or solvents are additionally added in step (b) of the process as defined below.

The stereogenic center marked with an * is either in (R)- or (S)-configuration at marked center, or it is in racemic form.

### Detailed Description of the Invention

### Process for the preparation of an intermediate of formula I

According to one aspect the present invention relates to a process for the preparation of an intermediate of formula I 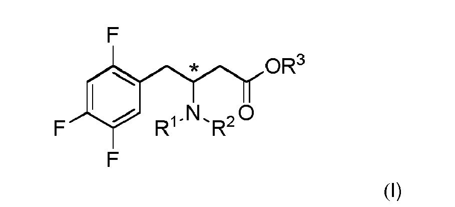 wherein R¹ and R² are identical or different, and are independently selected from
(i) hydrogen;
(ii) optionally chiral alkyl residues, having from 1 to 12 carbon atoms, wherein the alkyl residues are optionally aryl and/or aryloxy substituted;
(iii) optionally chiral alkyloxy residues, having from 1 to 12 carbon atoms, wherein the alkyloxy residues are optionally aryl substituted;
(iv) optionally chiral aryl residues, having from 6 to 24 carbon atoms, wherein the aryl residues are optionally alkyl and/or alkyloxy substituted;
(v) optionally chiral aryloxy residues, having from 6 to 24 carbon atoms, wherein the aryloxy residues are optionally alkyl substituted;
(vi) benzyl;
(vii) optionally chiral alkaloyl residues, having from 2 to 13 carbon atoms, wherein the alkaloyl residues are optionally aryl substituted;
(viii) optionally chiral aroyl residues, having from 7 to 25 carbon atoms, wherein the aryloxy residues are optionally alkyl substituted;
(ix) optionally chiral alkoxycarbonyl residues, having from 2 to 13 carbon atoms;
(x) optionally chiral aryloxycarbonyl residues, having from 7 to 25 carbon atoms;
(xi) tosyl;
(xii) optionally chiral silyl residues, having from 3 to 15 carbon atoms; and
(xiii) optionally chiral silyloxy residues, having from 3 to 15 carbon atoms;
   wherein R³ is selected from alkyl residues having from 1 to 6 carbon atoms;
   the process comprising the steps of:
   (a) providing an intermediate of formula II, 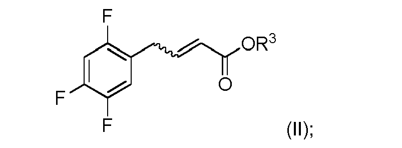
   (b) reacting the intermediate of formula II with an amine of formula III

      HNR¹R² (III)
wherein R¹ and R² are as defined above, in a protic solvent, particularly in water; or in a mixture of protic solvents, wherein the mixture particularly comprises water; or without adding of solvents in step (b); to obtain an intermediate of formula I.

In a preferred embodiment, in step (b) the solvent is selected from water, methanol, ethanol, iso-propanol, *tert-*butanol, trifluoroethanol, hexafluoro-2-propanol, amyl alcohol and any combination thereof, and is particularly water.

In another preferred embodiment step (b) is carried out without adding solvents.

In another preferred embodiment, step (b) is carried out at a temperature of 20 °C to 100 °C, preferably of 20 °C to 85 °C.

In a particularly preferred embodiment, R¹ and R² are identical, and are selected from
(i) hydrogen;
(ii) optionally chiral alkyl residues, having from 1 to 12 carbon atoms, wherein the alkyl residues are optionally aryl and/or aryloxy substituted;
(iii) optionally chiral aryl residues, having from 6 to 24 carbon atoms, wherein the aryl residues are optionally alkyl and/or alkyloxy substituted;
(iv) benzyl;
(v) optionally chiral alkoxycarbonyl residues, having from 2 to 13 carbon atoms;
(vi) optionally chiral aryloxycarbonyl residues, having from 7 to 25 carbon atoms;
(vii) optionally chiral silyl residues, having from 3 to 15 carbon atoms.

In a particularly preferred embodiment, R¹ and R² are hydrogen. In another particularly preferred embodiment, R¹ and R² are methyl. In still another particularly preferred embodiment, R¹ and R² are *N*-α-methylbenzyl. In another particularly preferred embodiment R¹ and R² are trimethylsilyl.

In another particularly preferred embodiment, R¹ and R² are different, and are independently selected from
(i) hydrogen;
(ii) optionally chiral alkyl residues, having from 1 to 12 carbon atoms, wherein the alkyl residues are optionally aryl and/or aryloxy substituted;
(iii) optionally chiral alkyloxy residues, having from 1 to 12 carbon atoms, wherein the alkyloxy residues are optionally aryl substituted;
(iv) optionally chiral aryl residues, having from 6 to 24 carbon atoms, wherein the aryl residues are optionally alkyl and/or alkyloxy substituted;
(v) optionally chiral aryloxy residues, having from 6 to 24 carbon atoms, wherein the aryloxy residues are optionally alkyl substituted;
(vi) benzyl;
(vii) optionally chiral alkaloyl residues, having from 2 to 13 carbon atoms, wherein the alkaloyl residues are optionally aryl substituted;
(viii) optionally chiral aroyl residues, having from 7 to 25 carbon atoms, wherein the aryloxy residues are optionally alkyl substituted;
(ix) optionally chiral alkoxycarbonyl residues, having from 2 to 13 carbon atoms;
(x) optionally chiral aryloxycarbonyl residues, having from 7 to 25 carbon atoms; and
(xi) tosyl;
(xii) optionally chiral silyl residues, having from 3 to 15 carbon atoms; and
(xiii) optionally chiral silyloxy residues, having from 3 to 15 carbon atoms.

In a particularly preferred embodiment R¹ is hydrogen and R² is tosyl. In another particularly preferred embodiment R¹ is hydrogen and R² is benzyl. In a particularly preferred embodiment R¹ is hydrogen and R² is *N-*α-methylbenzyl. In still another particularly preferred embodiment R¹ is benzyl and R² is *N*-α-methylbenzyl. In another particularly preferred embodiment R¹ is benzyl and R² is *N*-benzyl-1-phenethyl. In a further particularly preferred embodiment R¹ is hydrogen and R² is *O*-benzyl or *O*-methyl. In another particularly preferred embodiment R¹ is hydrogen and R² is *tert*-butyl-oxy-carbonyl or benzyl-oxy-carbonyl. In a further particularly preferred embodiment R¹ is hydrogen and R² is methoxy-phenyl. In a further particularly preferred embodiment R¹ is hydrogen and R² is *O*-phenyl. In another particularly preferred embodiment R¹ is hydrogen and R² is *O-*trimethylsilyl.

The chiral aryl residues defined for the intermediate of formula I are selected from *N*-α-methylbenzyl, *N*-bis[α-methylbenzyl], *N*-α-ethyl-naphthyl, 2-methoxybenzyl-1-phenylethyl, 3,4-dimethoxybenzyl-1-phenylethyl, and *N-*benzyl-1-phenethyl.

In the intermediate of formula I and the intermediate of formula II, R³ is typically selected from methyl, ethyl, propyl, cyclopropyl, butyl, pentyl, hexyl, isopropyl, isopentyl, *tert-*butyl*,* and is particularly methyl. In a particularly preferred embodiment R³ of the intermediate of formula II is methyl (intermediate of formula IIa).

In particularly preferred embodiments, the intermediate of formula **l** is

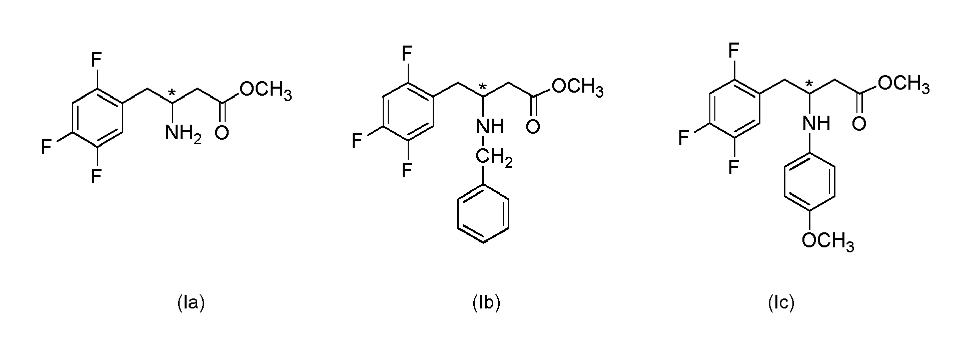

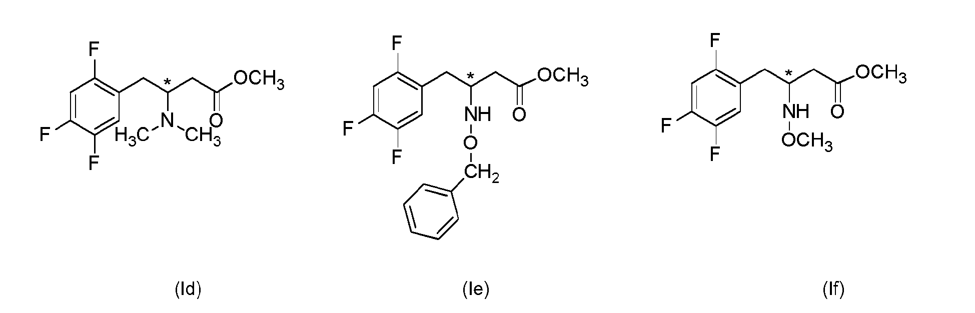

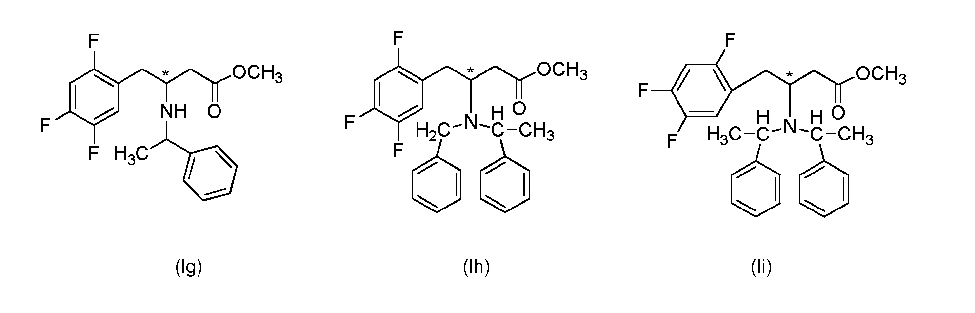

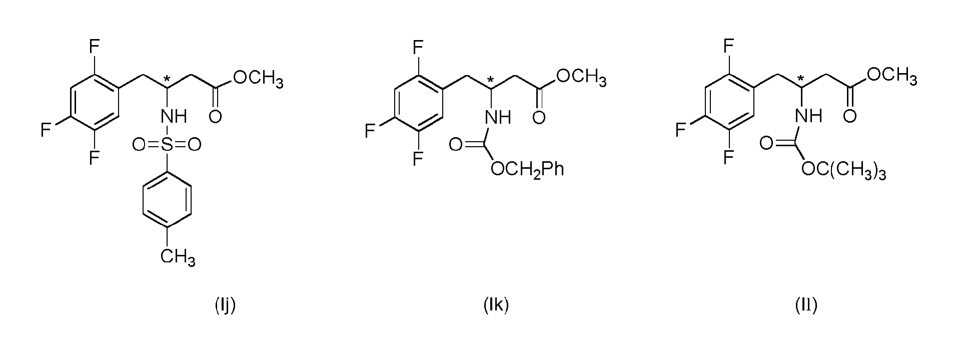

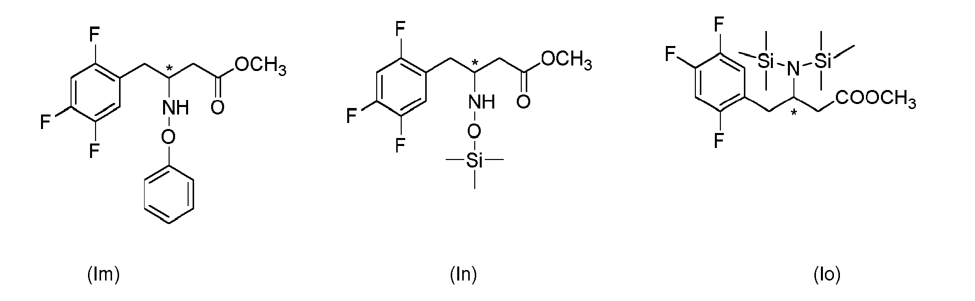

The amine of formula III is typically selected from ammonia, an alkyl amine, an aryl amine, an alkyl-aryl amine, a silyl amine, and a silyloxy amine.

Typically, when the amine is an alkyl amine it is selected from dimethylamine, *tert*-butyl-carbamate and O-methyl-hydroxylamine. Typically, when the amine is an aryl amine it is selected from benzylamine, p-methoxybenzylamine, 3,4-dimethoxybenzylamine, *p*-methoxyaniline, tosylamine, benzyl carbamate dibenzylamine, naphthylamine, *O*-benzyl-hydroxylamine, *O*-phenylhydroxylamine and benzhydrylamine. Typically, when the amine is an alkyl-aryl amine it is selected from methyl-phenyl-amine, *N*-α-methylbenzylamine, *N*-bis-[α-methylbenzylamine], and *N*-benzyl-1-phenylethyl. Typically, when the amine is a silyl amine it is selected from hexamethyldisilazane, potassium bis(trimethylsillyl)amide, sodium bis(trimethylsillyl)amide, lithium bis(trimethylsillyl)amide, 1,1,3,3-tetramethyldisilazane and 1,1,3,3-tetramethyl-1,3-diphenylsilazane. Typically, when the amine is a silyloxy amine it is selected from *O*-(trimethylsilyl)hydroxylamine, and *N,O-*bis(trimethylsillyl)hydroxylamine.

In preferred embodiment, the amine in step (b) is present in an amount of 1.0 to 2.0 equivalents, particularly 1.1 to 2.0, preferably about 1.2 to 1.7 equivalents, with respect to the intermediate of formula II.

In a further preferred embodiment, the reaction time in step (b) is between 5 to 52 hours, particularly between 6 to 48 hours.

In one embodiment, step (b) is a non-catalyzed process. Typically, when step (b) is carried out in a protic solvent or in a mixture of protic solvents, step (b) is then carried out at a temperature of 50 °C to 90 °C, preferably of 60 °C to 85 °C, and more preferably about 60 °C. Typically, when step (b) is carried out in a protic solvent or in a mixture of protic solvents, the reaction time is between 10 to 30 hours, preferably about 16 hours, when step (b) is a non-catalyzed process.

Typically, when step (b) is a non-catalyzed process, and when step (b) is carried out without adding of solvents, step (b) is then carried out at a temperature of 25 °C to 90 °C, preferably of 60 °C to 85 °C, and more preferably about 70 °C. Typically, when step (b) is carried out without adding of solvents, the reaction time is between 8 to 20 hours, preferably about 12 hours, when step (b) is a non-catalyzed process.

In another embodiment, when step (b) is carried out in a protic solvent or in a mixture of protic solvents, step (b) is a transition metal catalyzed process, particularly a transition metal catalyzed process using a catalyst comprising a transition metal compound, and optionally at least one ligand. Typically, the transition metal compound is selected from copper compounds, indium compounds, zinc compounds, iron compounds, manganese compounds, cerium compounds, bismuth compounds, scandium compounds, ytterbium compounds, yttrium(III) triflate, tin compounds and vanadium compounds, particularly selected from copper compounds, indium compounds, scandium compounds, ytterbium compounds and iron compounds. When the transition metal compound is a copper compound it is typically selected from copper(I) acetate, copper(I) chloride, copper(II) chloride, cooper(I) triflate, copper(II) triflate, cooper(II) acetylacetonate, cooper(II) chlorate, and any combination thereof, and is particularly selected from copper(I) acetate, copper(II) triflate, and copper(II) bromide. When the transition metal compound is an indium compound it is typically selected from indium(III) chloride, indium(II) chloride, indium(III) bromide, indium(III) perchlorate, and indium(III) nitrate, and is particularly indium(III) chloride. When the transition metal compound is a zinc compound it is typically selected from zinc(II) chloride, zinc(II) bromide, zinc(II) perchlorate, and zinc(II) oxalate, and is particularly zinc(II) chloride. When the transition metal compound is an iron compound it is typically selected from iron(II) chloride, iron(III) chloride, iron(II) acetylacetonate, iron(III) acetylacetonate, iron(III) chloride hexahydrate, iron(III) triflate, iron(III) chlorate, and iron(III) bromide, and is particularly iron(III) chloride. When the transition metal compound is a vanadium compound it is typically selected from vanadium(III) acetylacetonate, vanadium(V) oxychloride, vanadium(IV) chloride, and particularly vanadium(IV) chloride or oxychloride, and is particularly vanadium(III) acetylacetonate. When the transition metal compound is a scandium compound it is typically selected from scandium(III) triflate, scandium(III) oxalate, scandium(III) chloride, scandium(III) perchlorate, and is particularly scandium(III) triflate.

Typically, the transition metal compound is present in an amount of 2-25 mol%, particularly of 4-20 mol%, and more particularly about 8-15 mol% to the intermediate of formula II.

The optionally at least one ligand in the transition metal catalyzed process of step (b) is selected from monophosphine ligands, diphosphine ligands, and any combination thereof. The monophosphine ligand is typically selected from triphenylphosphine, tributylphosphine, trimethylphosphine, tricyclohexylphosphine, tri-(o-tolyl)-phosphine, tri-(2-furyl)phosphine, tris(dimethylamino)-phosphine, tribenzylphosphine, tripyrolydinophosphine, tris(4-methoxyphenyl)phosphine, and any combination thereof. The diphosphine ligand is typically selected from 1,2-bis(diphenyl-phosphino)benzene, 1,1,-bis(di-*tert*-butylphosphino)ferrocene, (oxydi-2,1-phenylene)bis-(diphenylphosphine), (*R*)-2,2-bis(diphenylphosphino)-1,1-binaphthalene, (S)- 2,2-bis(diphenylphosphino)-1,1-binaphthale, (*S*,*R*)-(diphenylphosphino)-ferrocenyl-ethyldi-*tert*-butylphosphine, (*R*,*S*)-(diphenylphosphino)-ferrocenyl-ethyldi-*tert*-butylphosphine, and any combination thereof. Typically, the at least one ligand is present in an amount of 2-20 mol%, particularly of 4-15 mol%, and more particularly 8-10 mol%, with respect to the intermediate of formula II.

In a preferred embodiment, the transition metal catalyzed process in step (b) is optionally carried out in the presence of a base, particularly wherein the base is selected from NaOBu*t*, KOBu*t*, K₂CO₃, Na₂CO₃, KOAc, NaOAc, and any combination thereof, more particularly NaOBu*t*. Typically, the base is present in an amount of 5-25 mol%, particularly of 10-20 mol%, and more particularly about 15 mol%, with respect to the intermediate of formula II.

In another preferred embodiment, the transition metal catalyzed process in step (b) is carried out in the absence of a base.

In a particularly preferred embodiment, the transition metal catalyzed process in step (b) is carried out in the presence of a surfactant as defined below.

In another embodiment, step (b) is an acid catalyzed process. In a preferred embodiment, the acid is a Lewis acid, particularly selected from copper(II) acetate, copper(II) chloride, copper(II) triflate, iron(III) chloride, indium(III) chloride, zinc(II) chloride, scandium(III) triflate, ytterbium(III) triflate and vanadium(III) acetyacetonate. In another preferred embodiment, the acid is a Brønsted acid, particularly selected from dodecylbenzenesulphonic acid (DBSA), phosphotungstic acid, phosphomolybdic acid, Nafion-H, trifluoromethanesulphonic acid (HOTf), methanesulphonic acid, *p*-toluenesulphonic acid (PTSA), chlorsulphonic acid, 2,5-dinitrobenzenesulphonic acid (DNBSA), sulphuric acid, polystyrenesulfonic acid (PSSA), boric acid, phenylboric acid, and any combination thereof, and is particularly DBSA, PSSA or phosphotungstic acid.

Typically, when step (b) is carried out in a protic solvent or in a mixture of protic solvents, and step (b) is an acid catalyzed process the acid then is present in an amount of 5-30 mol%, particularly of 8-25 mol%, and more particularly about 10-20 mol%, to the intermediate of formula II. Typically, when step (b) is carried out in a protic solvent or in a mixture of protic solvents, and step (b) is an acid catalyzed process step (b) is then carried out at a temperature of 25 °C to 90 °C, preferably of 60 °C to 85 °C, and more preferably about 60 °C to 65 °C. The reaction time is typically then between 6 to 24 hours, particularly between 10 to 24 hours.

Typically, when step (b) is carried out without adding of solvents, and step (b) is an acid catalyzed process the acid then is present in an amount of 3-30 mol%, particularly of 4-25 mol%, and more particularly about 5-20 mol% to the intermediate of formula II. Typically, when step (b) is carried out without adding of solvents, and step (b) is an acid catalyzed process step (b) is then carried out at a temperature of 20 °C to 90 °C, preferably of 20 °C to 60 °C. The reaction time is typically then between 10 to 52 hours, particularly between 12 to 48 hours.

In still another embodiment, when step (b) is carried out in a protic solvent or in a mixture of protic solvents, step (b) is an organocatalyzed process, particularly an organocatalyzed process using an optionally chiral organocatalyst. Typically, the organocatalyst is selected from amino acid chiral compounds, particularly selected from pyroglutamic acid, threonine, aspartic acid, and any combination thereof; proline derivatives; imidazolidinone derivatives; cinchona alkaloids; and tiourea derivatives. The organocatalyst is typically present in an amount of 1-30 mol%, particularly of 3-25 mol%, and more particularly 6-20 mol%, to the intermediate of formula II. The reaction time is typically between 12 to 24 hours, particularly between 15 to 20 hours, when step (b) is an organocatalyzed process. Typically, when step (b) is an organocatalyzed process, step (b) is then carried out at a temperature of 25 °C to 90 °C, preferably of 60 °C to 85 °C, and more preferably 60 °C to 80 °C.

In a further embodiment, when step (b) is carried out in a protic solvent or in a mixture of protic solvents, step (b) is carried out in the presence of a promoter. Typically, the promoter is an organic promoter. Typically, the promoter is selected from fluorinated alcohols, in particular selected from trifluoroethanol, hexafluoro-2-propanol, and any combination thereof.

The promoter is typically present in an amount of 1-15 equivalents, particularly of 3-12 equivalents, and more particularly 5-10 equivalents, to the intermediate of formula II. When step (b) is carried out in the presence of a promoter, step (b) is typically carried out at a temperature of 25 °C to 90 °C, preferably of 60 °C to 85 °C, and more preferably 60 °C to 80 °C. The reaction time is typically between 12 to 30 hours, particularly between 15 to 24 hours.

In still another embodiment, step (b) is carried out in the presence of a surfactant. Typically, the surfactant is selected from ionic, nonionic surfactants, and the combination thereof. When the surfactant is an ionic surfactant it is typically selected from sodium dodecyl sulfate, sodium stearate, sodium *N*-lauroylsarcosinate, cetyltrimethylammonium bromide, cetyltrimethylammonium chloride, benzyldodecyammonium bromide, and any combination thereof, and is particularly sodium dodecyl sulfate or cetyltrimethylammonium bromide. When the surfactant is a nonionic surfactant it is typically selected from D-α-tocopherol polyethylene glycol succinate, 4-octylphenol polyethoxylate, polyoxyethylene sorbitan monolaurate, polyethylene glycol dodecyl ether, and polyoxyethanyl-α-tocopheryl sebacate, and any combination thereof, and is particularly D-α-tocopherol polyethylene glycol succinate or polyoxyethanyl-α-tocopheryl sebacate.

When step (b) is carried out in a protic solvent or in a mixture of protic solvents, and step (b) is carried out in the presence of a surfactant, the surfactant is typically present in an amount of 0.5-30 wt%, particularly of 1-20 wt%, and more particularly 2-15 wt%, with respect to the intermediate of formula II. Typically, step (b) is carried out at a temperature of 25 °C to 90 °C, preferably of 60 °C to 85 °C, and more preferably 60 °C to 65 °C, when it is carried out in the presence of a surfactant and in a protic solvent or in a mixture of protic solvents. Typically, the reaction time is then between 10 to 20 hours, more particularly between 15 to 20 hours.

When step (b) is carried out without adding of solvents, and step (b) is carried out in the presence of a surfactant, the surfactant is typically present in an amount of 5-40 wt%, particularly of 10-30 wt%, and more particularly 15-20 wt% with respect to the intermediate of formula II. Typically, step (b) is carried out at a temperature of 25 °C to 90 °C, preferably of 60 °C to 85 °C, and more preferably 60 °C to 65 °C, when it is carried out in the presence of a surfactant and without adding of solvents in the step (b). Typically, the reaction time is then between 10 to 20 hours, more particularly between 16 to 18 hours.

In a particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 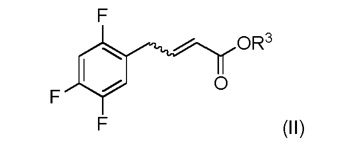
(b) reacting the intermediate of formula II with benzylamine present in an amount of 1.1 to 1.5 equivalents, preferably about 1.2 equivalents, with respect to the intermediate of formula II, in water at 20 °C to 65 °C, preferably at 60 °C, for 10 to 30 hours, preferably 24 hours, to obtain an intermediate of formula I.

In another particularly preferred embodiment in the process to obtain the intermediate of formula I, R¹ is hydrogen, R² is benzyl and R³ is methyl; and the process comprises or consists the steps of:
(a) providing an intermediate of formula II, wherein R³ is methyl; 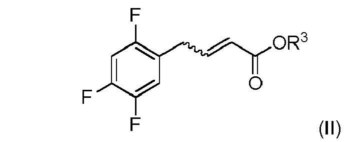
(b) reacting the intermediate of formula II with benzylamine present in an amount of 1.1 to 1.5 equivalents, preferably about 1.2 equivalents, with respect to the intermediate of formula II, in water at 30 °C for 5 to 7 hours, preferably 6 hours,
   in the presence of
   (i) copper(II) acetate, preferably present in an amount of 4-12 mol%, more preferably about 10 mol%, with respect to the intermediate of formula II;
   (ii) triphenylphosphine, preferably present in an amount of 4-12 mol%, more preferably 8-10 mol%, with respect to the intermediate of formula II,
   (iii) sodium dodecylsulfate, preferably present in an amount of 4-20 mol%, more preferably 8-10 mol%, with respect to the intermediate of formula II, and
   (iv) NaOBu*t*, preferably present in an amount of 10-20 mol%, more preferably about 15 mol%, with respect to the intermediate of formula II,
   to obtain an intermediate of formula I.

In a further particularly preferred embodiment in the process to obtain the intermediate of formula I, R¹ is
hydrogen, R² is benzyl and R³ is methyl; and
the process comprises or consists the steps of:
(a) providing an intermediate of formula II, wherein R³ is methyl; 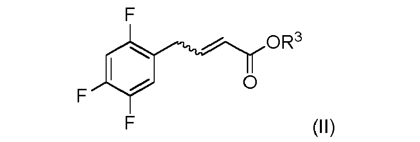
(b) reacting the intermediate of formula II with benzylamine present in an amount of 1.1 to 1.5 equivalents, preferably about 1.2 equivalents, with respect to the intermediate of formula II, in water at 30 °C to 65 °C, preferably at 60 °C, for 5 to 7 hours, preferably 6 hours,
   in the presence of
   (i) copper(II) acetate, preferably present in an amount of 4-12 mol%, more preferably about 10 mol%, with respect to the intermediate of formula II;
   (ii) sodium dodecylsulfate, preferably present in an amount of 4-20 mol%, more preferably 8-10 mol%, with respect to the intermediate of formula II, and
   to obtain an intermediate of formula I.

In another particularly preferred embodiment in the process to obtain the intermediate of formula I, R¹ is
hydrogen, R² is benzyl and R³ is methyl; and
the process comprises or consists the steps of:
(a) providing an intermediate of formula II, wherein R³ is methyl; 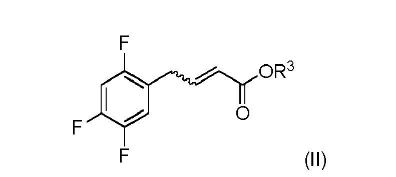
(b) reacting the intermediate of formula II with benzylamine present in an amount of 1.1 to 1.5 equivalents, preferably about 1.2 equivalents, with respect to the intermediate of formula II, in water at 50 °C to 95 °C, preferably at 80 °C, for 10 to 20 hours, preferably 15 hours,
   in the presence of
   (i) copper(II) bromide, preferably present in an amount of 10-20 mol%, more preferably about 15 mol%, with respect to the intermediate of formula II; or
   (ii) iron(III) chloride, preferably present in an amount of 10-20 mol%, more preferably about 15 mol%, with respect to the intermediate of formula II;
   to obtain an intermediate of formula I.

In still another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 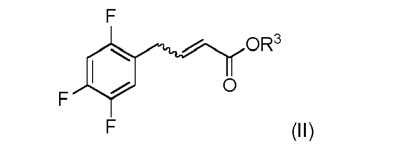
(b) reacting the intermediate of formula II with benzylamine present in an amount of 1.1 to 1.5 equivalents, preferably about 1.2 equivalents, with respect to the intermediate of formula II, in water at 25 °C to 90 °C, preferably at 60 °C, for 15 to 25 hours, preferably 20 hours,
in the presence of
indium(III) chloride, preferably present in an amount of 10-20 mol%, more preferably about 15 mol%, with respect to the intermediate of formula II;
to obtain an intermediate of formula I.

In another particularly preferred embodiment in the process to obtain the intermediate of formula I, R¹ is
hydrogen, R² is benzyl and R³ is methyl; and
the process comprises or consists the steps of:
(a) providing an intermediate of formula II, wherein R³ is methyl; 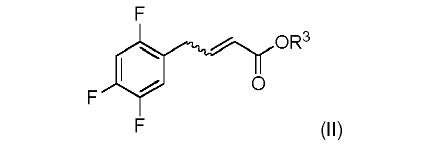
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 80 °C, for 10 to 20 hours, preferably 16 hours,
   in the presence of
   (i) 2,2,2-trifluoroethanol, preferably present in an amount of 1-15 equivalents and more preferably 5-10 equivalents with respect to the intermediate of formula II;
   to obtain an intermediate of formula I.

In yet another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 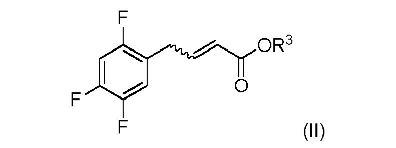
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 15 to 25 hours, preferably 20 hours,
   in the presence of
   (i) 1,1,1,3,3,3-hexafluoro-2-propanol, preferably present in an amount of 1-15 equivalents, more preferably 5-10 equivalents, with respect to the intermediate of formula II;
   to obtain an intermediate of formula I.

In another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 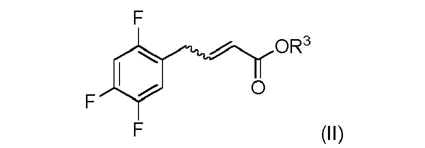
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 20 hours, preferably 15 hours,
   in the presence of
   (i) D-α-tocopherol-polyethyleneglycol-succinate, preferably present in an amount of 2-15 wt% with respect to the intermediate of formula II; or
   (ii) polyoxyethanyl-α-tocopheryl-sebacate, preferably present in an amount of 2-15 wt% with respect to the intermediate of formula II;
   to obtain an intermediate of formula I.

In a further particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 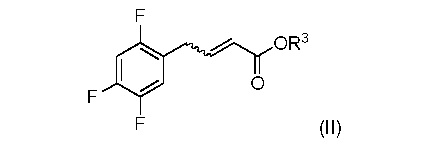
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 60 °C, for 15 to 25 hours, preferably 20 hours,
in the presence of
(i) sodium dodecylsulfate, preferably present in an amount of 2-15 wt% with respect to the intermediate of formula II;
   to obtain an intermediate of formula I.

In still another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 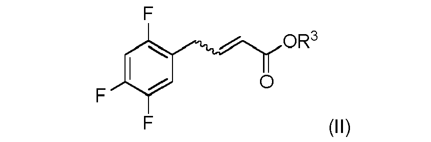
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 5 to 20 hours, preferably 12 hours,
   in the presence of
   (i) 4-dodecylbenzenesulfonic acid (DBSA), preferably present in an amount of 15-25 mol%, more preferably about 20 mol%, with respect to the intermediate of formula II;
      to obtain an intermediate of formula I.

In another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 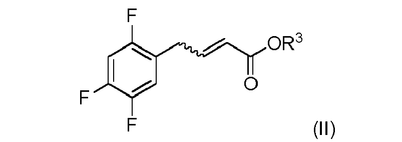
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 15 hours, preferably 20 hours, in the presence of
   (i) polystyrenesulfonic acid (PSSA), preferably present in an amount of 5-15 mol%, more preferably about 10 mol%, with respect to the intermediate of formula II;
      to obtain an intermediate of formula I.

In a further particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 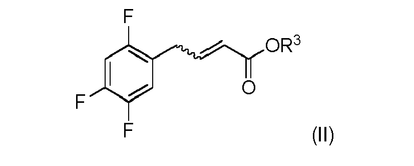
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 25 hours, preferably 15 hours,
   in the presence of
   (i) phosphotungstic acid, preferably present in an amount of 5-15 mol%, more preferably about 10 mol%, with respect to the intermediate of formula II;
      to obtain an intermediate of formula I.

In yet another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 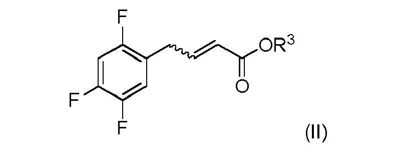
(b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 25 hours, preferably 18 hours,
   in the presence of
   (i) acid activator Nafion NR50, preferably present in an amount of 5-15 mol%, more preferably about 10 mol%, with respect to the intermediate of formula II;
      to obtain an intermediate of formula I.

In a further particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 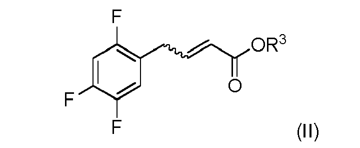
(b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 70 °C, for 8 to 20 hours, preferably 12 hours, to obtain an intermediate of formula I.

In another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 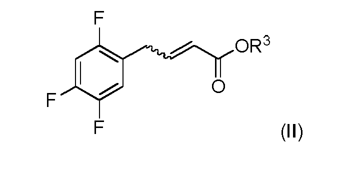
(b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 50 °C, preferably at ambient temperature, for 24 to 52 hours, preferably 48 hours,
   in the presence of
   (i) 4-dodecylbenzenesulfonic acid (DBSA), preferably present in an amount of 15-25 mol%, more preferably about 20 mol%, with respect to the intermediate of formula II; to obtain an intermediate of formula I.

In yet another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 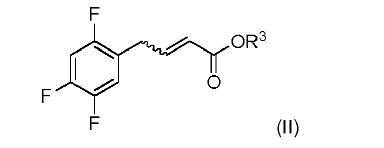
(b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 60 °C, for 10 to 25 hours, preferably 18 hours,
   in the presence of
   (i) phosphotungstic acid, preferably present in an amount of 3-10 mol%, more preferably about 5 mol%, with respect to the intermediate of formula II;
      to obtain an intermediate of formula I.

In a further particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 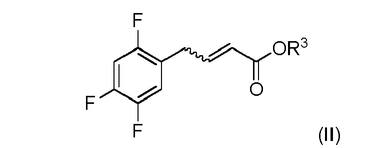
(b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 60 °C, for 10 to 48 hours, preferably 16 hours, in the presence of
   (i) sodium dodecylsulfate preferably present in an amount of 10-30 mol%, more preferably about 20 mol%, with respect to the intermediate of formula II,
      to obtain an intermediate of formula I.

In another particularly preferred embodiment in the process to obtain the intermediate of formula I, R¹ is hydrogen, R² is benzyl and R³ is methyl; and
the process comprises or consists the steps of:
(a) providing an intermediate of formula II, wherein R³ is methyl; 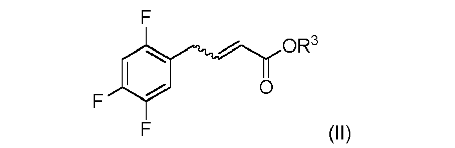
(b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 60 °C, for 10 to 48 hours, preferably 18 hours,
   in the presence of
   (i) cetyltrimethylammonium bromide, preferably present in an amount of 10-30 mol%, more preferably about 20 mol%, with respect to the intermediate of formula II,
      to obtain an intermediate of formula I.

In a further particularly preferred embodiment in the process to obtain the intermediate of formula I, R¹ is hydrogen, R² is *O*-benzyl and R³ is methyl; and
the process comprises or consists the steps of:
(a) providing an intermediate of formula II, wherein R³ is methyl; 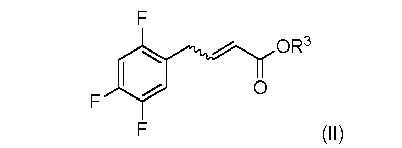
(b) reacting the intermediate of formula II with *O*-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
   in the presence of
   (i) 4-dodecylbenzenesulfonic acid (DBSA), preferably present in an amount of 15-25 mol%, more preferably about 20 mol%, with respect to the intermediate of formula II;
      to obtain an intermediate of formula I.

In another particularly preferred embodiment in the process to obtain the intermediate of formula I, R¹ is hydrogen, R² is *O*-benzyl and R³ is methyl; and
the process comprises or consists the steps of:
(a) providing an intermediate of formula II, wherein R³ is methyl; 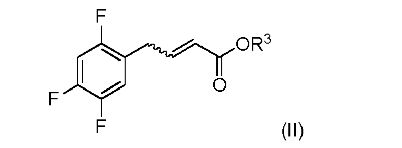
(b) reacting the intermediate of formula II with *O*-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
   in the presence of
   (i) 2,2,2-trifluoroethanol, preferably present in an amount of 1-15 equivalents, preferably 3-12 equivalents, and more preferably 5 equivalents with respect to the intermediate of formula II;
      to obtain an intermediate of formula I.

In another particularly preferred embodiment in the process to obtain the intermediate of formula I, R¹ is hydrogen, R² is *O*-benzyl and R³ is methyl; and
the process comprises or consists the steps of:
(a) providing an intermediate of formula II, wherein R³ is methyl; 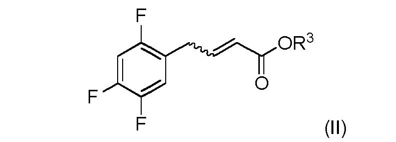
(b) reacting the intermediate of formula II with *O*-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
   in the presence of
   (i) scandium(III) triflate, present in an amount of 1-30 mo%, preferably about 5-25 mol%, more preferably about 20 mol%, with respect to the intermediate of formula II; and
   (ii) sodium dodecylsulfate, present in an amount of 1-30 mol%, preferably about 5-25 mol%, more preferably about 20 mol%, with respect to the intermediate of II;
      to obtain an intermediate of formula I.

In another particularly preferred embodiment in the process to obtain the intermediate of formula I, R¹ is hydrogen, R² is *O*-benzyl and R³ is methyl; and
the process comprises or consists the steps of:
(a) providing an intermediate of formula II, wherein R³ is methyl; 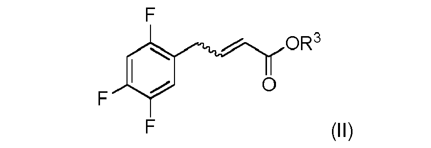
(b) reacting the intermediate of formula II with *O*-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
   in the presence of
   (i) 2,2,2-trifluoroethanol, present in an amount of 1-15 equivalents, preferably about 3-12 equivalents, more preferably about 5 equivalents, with respect to the intermediate of formula II; and
   (ii) sodium dodecylsulfate, preferably present in an amount of 1-30 mol%, preferably about 5-20 mol%, more preferably about 8 mol%, with respect to the intermediate of II; to obtain an intermediate of formula I.

According to another aspect, there is also provided the use of a process as defined above to obtain an intermediate of formula I in a process for the preparation of anti-diabetic agents, in particular (*R*)-3-Amino-1-[3-(trifluormethyl)-5,6,7,8-tetrahydro[1,2,4]triazol[4,3-*a*]pyrazin-7-yl]- 4-(2,4,5-trifluorphenyl)butan-1-on.

The invention will be more fully understood by references to the following examples. They should not, however, be construed as limiting the scope of the invention. The disclosure of all literature and patent citations mentioned herein are expressly incorporated by reference.

### EXAMPLES

### Example 1: Synthesis of methyl (E)-4-(2,4,5-trifluorophenyl)-but-2-enoate (IIa) by cobalt catalyzed cross-coupling process:

A dry and nitrogen-flushed 200 mL two-necked flask, equipped with a magnetic stirrer and a rubber septum was charged with anhydrous THF (20 mL) and cooled to -20 °C. Afterwards 2,4,5-trifluorobenzene (65,2 mmol, 13.7 g, 7.6 mL) was iniciated through a septum following by very slow addition of *i*PrMgCl (2 M in THF, 1.2 equiv. according to 2,4,5-trifluorobenzene, 39.6 mL). The reaction temperature was maintained at -10 °C and the reaction mixture was stirred for an hour, until Br/Mg exchange reaction was completed and 2,4,5-trifluroarylmagnesium bromide (chloride) was formed.

Into another three-necked dry flask flushed with nitrogen, were placed CoBr₂ (3.76 mmol, 6 mol%, 822 mg, 99.99% purity), TMEDA (3.76 mmol, 6 mol%, 564 mL) and anhydrous THF (20 mL). Such reaction system was cooled to 0 °C and during intensive stirring methyl *trans*-4-bromo-2-butenoate (50 mmol, 8.95 g, 5.98 ml, 90% purity) was iniciated through a rubber septum and reaction mixture was stirred for 30 min. Finally, freshly prepared THF solution of Grignard reagent 2,4,5-trifluoroarylmagnesium bromide (chloride) previously cooled to - 20 °C, was slowly added and such reaction mixture was intensively stirred at 0 °C for 10-16 hours. The saturated aqueous NH₄Cl solution (150 mL) was added and reaction mixture was gently extracted with four portions of EtOAc (300 mL). The combined organic phases were washed with brine (200 mL), dried over anhydrous Mg₂SO₄ and solvent was evaporated under reduced pressure. The crude residue was purified with column chromatography (Isolera; gradient elution *n*-hexane/EtOAc = 1/10) to obtain pure liquid/oily product (**IIa**) (10.8 g, 93%) as determined with ¹H, ¹⁹F and ¹³C NMR analysis.
¹H NMR (500 MHz, CDCl3, ppm) δ 6.90-7.05 (m, 2ArH + 1H), 5.80 (dt, J = 15.5 Hz, J= 1.5 Hz 1H), 3.73 (s, 3H), 3.50 (d, J = 6.6 Hz, 2H).
¹³C NMR (125 MHz, CDCl₃, ppm) δ 30.6, 51.4, 105.5 (dd, J = 28.5 Hz, J = 21.5 Hz), 118.1 (dd, J = 19.0 Hz, J = 6.0 Hz), 120.8 (m), 124.5, 145.5, 147.8 (m), 150.1 (m), 156.8 (m), 166.3 (C=O).
¹⁹F NMR (470 MHz, CDCl₃, ppm) δ -120.4 (m), -136.3 (m), -143.5 (m).

### Example 2: Synthesis of methyl 3-benzylamino-4-(2,4,5-trifluorophenyl)butanoate (Ib) from (IIa) through aza-Michael reaction in pure water:

(β-unsaturated ester methyl (*E*)-4-(2,4,5-trifluorophenyl)-but-2-enoate (**IIa**) (0.5 mmol, 115 mg) and benzylamine (0.6 mmol, 68 mg, 99% purity) were placed into a glass flask and the deionized water was added (3 mL). The heterogenic reaction mixture (aqueous dispersion) was intensively stirred (1000 rpm) at 60 °C for 16 hours. The reaction mixture was diluted with water (5 mL) and gently extracted with EtOAc (2 x 25 mL). The combined organic layers were dried over anhydrous Mg₂SO₄, the organic solvent was evaporated under reduced pressure and obtained crude reaction mixture (165 mg) was analyzed with ¹H,NMR spectroscopy. The crude product (**Ib**) was purified using column chromatography (SiO₂, hexane:ethyacetate = 2 :1) and brownish liquid product (I**b**) (102 mg, 61% yield) was obtained. The compound methyl 3-benzylamino-4-(2,4,5-trifluorophenyl)butanoate (**Ib**) was confirmed also with ¹³C NMR analysis and ¹H-¹⁵N HMBC NMR correlation spectroscopy.
¹H NMR (500 MHz, CDCl₃, ppm) δ 7.28 (m, 5H), 7.04 (m, 1H), 6.90 (m, 1H), 3.84 (dd, J = 6.5 Hz, J = 2.2 Hz, 2H), 3.67 (s, 3H), 3.25 (pentet, J = 6.3 Hz, 1H_{c}, H**_{c}**C-NH-), 2.90 (dd, J = 14.0 Hz, J = 6.3 Hz, 1Ha, HₐC-COO-), 2.72 (dd, J = 14 Hz, J = 6.3 Hz, 1H_{b}, H_{b}C-COO-), 2.45 (dd, J = 21.0 Hz, J = 6.3 Hz, 2H), 1.95 (bs, NH).
¹³C NMR (125 MHz, CDCl₃, ppm) δ 172.3, 156.1 (dd, J = 235.0 Hz, J = 1.25 Hz), 148.5 (dd, J = 247.0 Hz, J = 1.25 Hz), 146.5 (dd, J = 267 Hz, J = 15 Hz), 140.0, 128.4, 128.0, 127.0, 122.1, 119.1 (dd, J = 18.75 Hz, J = 5.0 Hz), 105.3 (dd, J = 28.75 Hz, J = 21.25 Hz), 54.4, 51.6, 51.0, 38.4, 33.0.

### Example 3: Copper-catalyzed [Cu(OAc)₂] synthesis of methyl 3-benzylamino-4-(2,4,5-trifluorophenyl)butanoate (Ib) from (IIa) through aza-Michael reaction in water in the presence of phosphine ligand and surfactant:

In a two-necked round bottom flask were placed Cu(OAc)₂ (0.11 mmol, 19.1 mg), NaOBu*t* (0.13 mmol, 12.6 mg), Ph₃P (0.11 mmol, 28.8 mg) and anionic surfactant sodium dodecylsulfate (SDS) (0.042 mmol, 12 mg) under the nitrogen. Afterwards the deionized water (3 mL) was added and the reaction mixture was vigorously stirred (900 rpm) at ambient temperature for 30 min. Than the dispersion of β -unsaturated ester methyl (*E*)-4-(2.4.5-trifluorophenyl)-but-2-enoate (**IIa**) (1 mmol) in 2 mL of deionized water was slowly added through the rubber septum into the aqueous micellar solution (final volume: 5 mL of 0.0081 M SDS aqueous solution) following by addition of benzylamine (1 mmol, 107 mg, 110 µL). Such aqueous reaction system was intensively stirred (900 rpm) at ambient temperature for 6 hours. The reaction mixture was diluted with water (5 mL), gently extracted with EtOAc (2 x 25 mL), the combined organic layers were dried over anhydrous Mg₂SO₄ and solvent was evaporated under reduced pressure. The obtained crude reaction mixture was analyzed with ¹H NMR and purified with column chromatography (SiO₂, hexane:ethyl acetate = 2 : 1) to obtain (200 mg, 60% yield). The compound methyl 3-benzylamino-4-(2,4,5-trifluorophenyl)butanoate (**Ib**) was confirmed also with HPLC-MS analysis.

### Example 4: Copper-catalyzed [CuBr₂] synthesis of methyl 3-benzylamino-4-(2,4,5-trifluorophenyl)butanoate (Ib) from (IIa) through aza-Michael reaction in water with no presence of any ligand and base:

In a glass round bottom flask equipped with magnetic stirrer and condenser were placed CuBr₂ (0.075 mmol; 15 mol% according to (**IIa**), 17 mg), methyl (*E*)-4-(2,4,5-trifluorophenyl)-but-2-enoate (**IIa**) (0.5 mmol, 115 mg) and were well suspended in 2.5 mL of water (800 rpm). Such aqueous system was slowly heated to 80 °C, than benzylamine (0.6 mmol; 1.2 equiv. according to (**IIa**), 66 µL) was dropped into reaction system and vigorously stirred for 15 hours. The reaction mixture was diluted with water (2.5 mL), gently extracted with EtOAc (2 x 25 mL), the combined organic layers were washed than with brine (1 x 30 mL) and finally dried over anhydrous Mg₂SO₄. After the solvent was evaporated under reduced pressure, the obtained crude product (**Ib**) (154 mg; 91.5 % yield) was analyzed with ¹H NMR analysis. The compound methyl 3-benzylamino-4-(2,4,5-trifluorophenyl)butanoate (**Ib**) was confirmed also with HPLC-MS analysis.

### Example 5: Iron(III) chloride catalyzed synthesis of methyl 3-benzylamino-4-(2,4,5-trifluorophenyl)butanoate (lb) from (IIa) through aza-Michael reaction in water :

In a thick-walled glass vial equipped with an magnetic stir bar were placed FeCl₃ x 6H₂O (0.075 mmol; 15 mol% according to (**IIa**), 21 mg), methyl (*E*)-4-(2,4,5-trifluorophenyl)-but-2-enoate (**IIa**) (0.5 mmol, 115 mg) and were well suspended in 2.5 mL of water (800 rpm). Such aqueous system was slowly heated to 80 °C, than benzylamine (0.6 mmol; 1.2 equiv. according to (**IIa**), 66 µL) was dropped into reaction system which was than vigorously stirred for 15 hours. The reaction mixture was diluted with water (2.5 mL), gently extracted with EtOAc (2 x 25 mL), the combined organic layers were washed than with brine (1 x 30 mL) and finally dried over anhydrous Mg₂SO₄. After the solvent was evaporated under reduced pressure, the obtained crude product (**Ib**) (140 mg; 83 % yield) was analyzed with ¹H NMR analysis. The compound methyl 3-benzylamino-4-(2,4,5-trifluorophenyl)butanoate (**Ib**) was confirmed also with HPLC-MS analysis.

### Example 6: Indium(III) chloride-catalyzed synthesis of methyl 3-benzylamino-4-(2,4,5-trifluorophenyl)butanoate (lb) From (IIa) through aza-Michael reaction in water :

In a thick-walled glass vial equipped with an magnetic stir bar were placed InCl₃ (0.075 mmol; 15 mol% according to **(IIa),** 16.7 mg), methyl (*E*)-4-(2,4,5-trifluorophenyl)-but-2-enoate (**IIa**) (0.5 mmol, 115 mg) and were well suspended in 2.5 mL of water (800 rpm). Such aqueous system was slowly heated to 60 °C, than benzylamine (0.6 mmol; 1.2 equiv. according to (**IIa**), 66 µL) was dropped into reaction system which was than vigorously stirred for 20 hours. The reaction mixture was diluted with water (2.5 mL), gently extracted with EtOAc (2 x 25 mL), the combined organic layers were washed than with brine (1 x 30 mL) and finally dried over anhydrous Mg₂SO₄. After the solvent was evaporated under reduced pressure, the obtained crude product (**Ib**) (150 mg; 89 % yield) was analyzed with ¹H NMR analysis. The compound methyl 3-benzylamino-4-(2,4,5-trifluorophenyl)butanoate (**Ib**) was confirmed also with HPLC-MS analysis.

### Example 7: Synthesis of methyl 3-benzylamino-4-(2,4,5-trifluorophenyl)butanoate (Ib) from (IIa) through aza-Michael reaction in water in the presence of 2,2,2-trifluoroethanol (TFE) as promoter:

In a thick-walled glass vial equipped with an magnetic stir bar was placed methyl (*E*)-4-(2,4,5-trifluorophenyl)-but-2-enoate (**IIa**) (0.5 mmol, 115 mg) which was than vigorously stirred in 2.0 mL of water (900 rpm) to obtain well dispersed aqueous system. Afterwards 2,2,2-trifluoroethanol (5 mmol, 10 equiv. according to (**IIa**), 360 µL) was slowly dropped into heterogenic reaction system and slowly heated to 80 °C. Finally benzylamine (0.6 mmol, 66 µL) was slowly added and such reaction mixture was vigorously stirred (900 rpm) at 80 °C for 16 hours. Solvent TFE was first evaporated under reduced pressure, organic aqueous residue was extracted with EtOAc (2 x 30 mL) and combined organic phases were finally washed with brine (1 x 40 mL). After the solvent was evaporated under reduced pressure, the obtained crude product (**Ib**) (154 mg; 91 % yield) was analyzed and determined with ¹H, ¹³C NMR analysis. The compound methyl 3-benzylamino-4-(2,4,5-trifluorophenyl)butanoate (**Ib)** was confirmed also with HPLC-MS analysis.

### Example 8: Synthesis of methyl 3-benzylamino-4-(2,4,5-trifluorophenyl)butanoate (Ib) from (IIa) through aza-Michael reaction in water in the presence of 1,1,1,3,3,3-hexafluoro-2-propanol (HFIP) as promoter:

In a thick-walled glass vial equipped with an magnetic stir bar was placed methyl (*E*)-4-(2,4,5-trifluorophenyl)-but-2-enoate (**IIa**) (0.5 mmol, 115 mg) which was than vigorously stirred in 2.0 mL of water (900 rpm) to obtain well dispersed aqueous system. Afterwards 1,1,1,3,3,3-hexafluoro-2-propanol (5 mmol, 10 equiv. according to (**IIa**), 526 µL) was slowly dropped into heterogenic reaction system and slowly heated to 65 °C. Finally benzylamine (0.6 mmol, 66 µL) was slowly added and such reaction mixture was vigorously stirred (900 rpm) at 65 °C for 20 hours. Solvent HFIP was first evaporated under reduced pressure, organic aqueous residue was extracted with EtOAc (2 x 30 mL) and combined organic phases were finally washed with brine (1 x 40 mL). After the solvent was evaporated under reduced pressure, the obtained crude product (**Ib**) (142 mg; 84 % yield) was analyzed and determined with ¹H, ¹³C NMR analysis. The compound methyl 3-benzylamino-4-(2,4,5-trifluorophenyl)butanoate (**Ib**) was confirmed also with HPLC-MS analysis.

### Example 9: Synthesis of methyl 3-benzylamino-4-(2,4,5-trifluorophenyl)butanoate (Ib) from (IIa) through aza-Michael reaction in water in the presence of non-ionic surfactant D-α-tocopherol-polyethyleneglycol-succinate (TPGS) (micelle-based aqueous system):

Starting material methyl (*E*)-4-(2,4,5-trifluorophenyl)-but-2-enoate (**IIa**) (0.5 mmol, 115 mg) was highly suspended in 3 mL 2 wt% aqueous solution of surfactant TPGS and such reaction mixture was vigorously stirred (800 rpm) for 20 minutes. Afterwards benzylamine (0.6 mmol, 1.2 equiv., 66 µL) was slowly dropped into aqueous system (3 µL/min) and reaction mixture was heated at 65 °C for 15 hours. The reaction mixture was diluted with brine (4 mL), gently extracted with EtOAc (2 x 30 mL) and combined organic layers were dried over anhydrous Mg₂S0₄. After the solvent was evaporated under reduced pressure, the obtained crude product (**Ib**) (150 mg; 89 % yield) was analyzed and proved with ¹H NMR analysis. The compound methyl 3-benzylamino-4-(2,4,5-trifluorophenyl)butanoate (**Ib**) was confirmed also with HPLC-MS analysis.

### Example 10: Synthesis of methyl 3-benzylamino-4-(2,4,5-trifluorophenyl)butanoate (Ib) from (IIa) through aza-Michael reaction in water in the presence of non-ionic surfactant polyoxyethanyl-α-tocopheryl-sebacate (PTS) (micelle-based aqueous system):

Starting material methyl- (*E*)-4-(2,4,5-triftuorophenyl)-but-2-enoate (**IIa**) (0.5 mmol, 115 mg) was highly suspended in 3 mL of aqueous solution of surfactant PTS (3 wt%) and such reaction mixture was vigorously stirred (800 rpm) for 20 minutes. Afterwards benzylamine (0.6 mmol, 1.2 equiv., 66 µL) was slowly dropped into aqueous system (3 µL/min) and reaction mixture was heated at 65 °C for 15 hours. The reaction mixture was diluted with brine (4 mL), gently extracted with EtOAc (2 x 30 mL) and combined organic layers were dried over anhydrous Mg₂S0₄. After the solvent was evaporated under reduced pressure, the obtained crude product (**Ib**) (143 mg; 85 % yield) was analyzed and confirmed with ¹H NMR analysis.

### Example 11: Synthesis of methyl 3-benzylamino-4-(2,4,5-trifluorophenyl)butanoate (Ib) from (IIa) through aza-Michael reaction in water in the presence of ionic surfactant sodium dodecysulfate (SDS) (micelle-based aqueous system):

In a thick-walled glass vial equipped with an magnetic stir bar was placed methyl (E)-4-(2,4,5-trifluorophenyl)-but-2-enoate (**IIa**) (0.5 mmol, 115 mg) and 5 mL of aqueous solution of SDS in its concentration 0.008 M was than added. The reaction system was vigorously stirred for 20 min and Afterwards benzylamine (0.6 mmol, 66 µL) was slowly added. Such aqueous mixture was heated at 60 °C during intense stirring (800 rpm) for 20 hours. The reaction mixture was diluted with water (5 mL), gently extracted with EtOAc (2 x 35 mL), the combined organic layers were dried over anhydrous Mg₂SO₄ and solvent was evaporated under reduced pressure. The obtained crude product (**Ib**) (148 mg, 88 % yield) was analyzed and confirmed with ¹H NMR.

### Example 12: Synthesis of methyl 3-benzylamino-4-(2,4,5-trifluorophenyl)butanoate (Ib) from (IIa) through aza-Michael reaction in water in the presence of surfactant-type Brønsted acid 4-dodecybenzenesulfonic acid (DBSA) (acidic micelle-based catalysis in water):

In a thick-walled glass vial equipped with an magnetic stir bar was placed DBSA (0.1 mmol, 20 mol% according to (**IIa**), 33 mg) and dissolved in 2.5 mL of water under intensive stirring. Afterwards methyl (E)-4-(2,4,5-trifluorophenyl)-but-2-enoate (**IIa**) (0.5 mmol, 115 mg) was added and reaction mixture was vigorously stirred for 10 min at 65 °C. Finally benzylamine (0.6 mmol, 66 µL) was slowly added and such aqueous micellar system was stirred (800 rpm) at 65 °C for 12 hours. Reaction mixture was diluted with saturated aqueous solution of NaHCO₃ (3.5 mL) and gently extracted with EtOAc (2 x 35 mL). The combined organic phases was finally washed with brine (1 x 40 mL), dried over anhydrous Na₂SO₄ and organic solvent was evaporated under reduced pressure. The obtained crude product (**Ib**) (154 mg, 91 % yield) was analyzed and confirmed with ¹H and ¹³C NMR analysis. The compound methyl 3-benzylamino-4-(2,4,5-trifluorophenyl)butanoate (**Ib**) was confirmed also with HPLC-MS analysis.

### Example 13: Synthesis of methyl 3-benzylamino-4-(2,4,5-trifluorophenyl)butanoate (Ib) from (IIa) through aza-Michael reaction in water in the presence of polystyrenesulfonic acid (PSSA) as Brønsted acid promoter:

In a thick-walled glass vial equipped with an magnetic stir bar was placed PSSA (0.05 mmol, 10 mol% according to (**IIa**)) and dissolved in 2.0 mL of water under intensive stirring. Afterwards methyl (E)-4-(2,4,5-trifluorophenyl)-but-2-enoate (**IIa**) (0.5 mmol, 115 mg) was added and reaction mixture was vigorously stirred (800 rpm) for 10 min at 70 °C. Finally benzylamine (0.6 mmol, 66 µL) was slowly added and such aqueous system was stirred (800 rpm) at 65 °C for 20 hours. Reaction mixture was diluted with saturated aqueous solution of NaHCO₃ (3.5 mL) and gently extracted with EtOAc (2 x 35 mL). The combined organic phases was finally washed with brine (1 x 40 mL), dried over anhydrous Na₂SO₄ and organic solvent was evaporated under reduced pressure. The obtained crude product (**Ib**) (141 mg, 83 % yield) was analyzed and confirmed with ¹H and ¹³C NMR and HPLC-MS analysis.

### Example 14: Synthesis of methyl 3-benzylamino-4-(2,4,5-trifluorophenyl)butanoate (Ib) from (IIa) through aza-Michael reaction in water in the presence of phosphotungstic acid:

In a thick-walled glass vial equipped with an magnetic stir bar was placed methyl (E)-4-(2,4,5-trifluorophenyl)-but-2-enoate (**IIa**) (0.5 mmol, 115 mg) and 3 mL of water was added. Then 12-phosphotungstic acid was added (0.05 mmol, 10 mol% according to (**IIa**)) and reaction mixture was intensively stirred (800 rpm) for 10 min at 60 °C. Finally benzylamine (0.6 mmol, 66 µL) was slowly dropped and such aqueous system was stirred (800 rpm) at 65 °C for 15 hours. Reaction mixture was diluted with saturated aqueous solution of NaHCO₃ (3.5 mL) and gently extracted with EtOAc (2 x 35 mL). The combined organic phases was finally washed with brine (1 x 40 mL), dried over anhydrous Na₂SO₄ and organic solvent was evaporated under reduced pressure. The obtained crude product (**Ib**) (135 mg, 80 % yield) was analyzed and confirmed with ¹H NMR and HPLC-MS analysis.

### Example 15: Synthesis of methyl 3-benzylamino-4-(2,4,5-trifluorophenyl)butanoate (Ib) from (IIa) through aza-Michael reaction in water in the presence of acid activator Nafion NR50 as a Reusable Catalyst:

In a thick-walled glass vial equipped with an magnetic stir bar was placed methyl (E)-4-(2,4,5-trifluorophenyl)-but-2-enoate (**IIa**) (0.5 mmol, 115 mg) and 2.5 mL of water was added. Than acid activator Nafion NR50 (0.05 mmol, 10 mol% according to (**IIa**)) was added and reaction mixture was intensively stirred (800 rpm) for 10 min at 70 °C. Finally benzylamine (0.6 mmol, 66 µL) was slowly dropped into aqueous system and the reaction mixture was stirred (800 rpm) at 65 °C for 18 hours. The catalyst Nafion NR50 was first gently filtered off, aqueous phase was than slowly extracted with two portions of EtOAc (30 ml) and finally washed with an aqueous solution of NaHCO₃. The combined organic phases were dried over anhydrous Na₂SO₄ and organic solvent was evaporated under reduced pressure. The obtained crude product (**Ib**) (143 mg, 85 % yield) was analyzed and confirmed with ¹H and ¹³C NMR and HPLC-MS analysis.

### Example 16: Synthesis of methyl 3-benzylamino-4-(2,4,5-trifluorophenyl)butanoate (Ib) from (IIa) through aza-Michael reaction without adding of solvents:

In a thick-walled glass vial equipped with an magnetic stir bar was placed (methyl (*E*)-4-(2,4,5-trifluorophenyl)-but-2-enoate (**IIa**) (0.5 mmol, 115 mg) and reaction system was slowly heated to 60 °C under intensive stirring. Afterwards benzylamine (0.6 mmol; 66 mL) was slowly added to (**IIa**) and such reaction system was vigorously stirred (800 rpm) at 70 °C for 12 hours. The reaction mixture was than extracted with *tert*-butyl methyl ether (20 mL), organic phase was washed with brine (20 mL) and dried over anhydrous Na₂SO₄. After evaporation of organic solvent under reduced pressure we obtained crude product (**Ib**) (85 mg, 50 % yield) which was analyzed and confirmed with ¹H and ¹³C NMR.

### Example 17: Synthesis of methyl 3-benzylamino-4-(2,4,5-trifluorophenyl)butanoate (Ib) from (IIa) through aza-Michael reaction without adding of solvents in the presence of catalytic amount of DBSA at ambient temperature:

In a thick-walled glass vial equipped with an magnetic stir bar were placed (methyl (*E*)-4-(2,4,5-trifluorophenyl)-but-2-enoate (**IIa**) (0.5 mmol, 115 mg), DBSA (0.1 mmol, 32 mg) and reaction system was vigorously stirred for 15 min at ambient temperature. Afterwards benzylamine (0.6 mmol; 66 mL) was slowly added and such reaction mixture was stirred (800 rpm) for 48 hours under ambient temperature. The reaction mixture was than extracted with *tert*-butyl methyl ether (20 mL), organic phase was washed with aqueous solution of NaHCO₃ (20 mL) and dried over anhydrous Na₂SO₄. After evaporation of organic solvent under reduced pressure we obtained crude product (**Ib**) (105 mg, 62 % yield) which was analyzed and confirmed with ¹H and ¹³C NMR.

### Example 18: Synthesis of methyl 3-benzylamino-4-(2,4,5-trifluorophenyl)butanoate (Ib) from (IIa) through aza-Michael reaction without adding of solvents in the presence of phosphotungstic acid:

In a thick-walled glass vial equipped with an magnetic stir bar was placed methyl (*E*)-4-(2,4,5-trifluorophenyl)-but-2-enoate (**IIa**) (0.5 mmol, 115 mg) and after that 12-phosphotungstic acid was added (0.025 mmol, 5 mol% according to (**IIa**)) and neat reaction mixture was intensively stirred (700 rpm) for 10 min at 60 °C. Afterwards benzylamine (0.6 mmol, 66 mL) was slowly dropped and such reaction system was stirred at 60 °C for 18 hours. Reaction mixture was diluted with saturated aqueous solution of NaCl (3.5 mL) and gently extracted with EtOAc (35 mL). The organic phase was dried over anhydrous Na₂SO₄ and solvent was evaporated under reduced pressure. The obtained crude product (**Ib**) (68 mg, 40 % yield) was analyzed and confirmed with ¹H NMR spectroscopy.

### Example 19: Synthesis of methyl 3-benzylamino-4-(2,4,5-trifluorophenyl)butanoate (Ib) from (IIa) through aza-Michael reaction without adding of solvents in the presence of sodium dodecysulfate (SDS):

In a thick-walled glass vial equipped with an magnetic stir bar was placed methyl (*E*)-4-(2,4,5-trifluorophenyl)-but-2-enoate (**IIa**) (0.5 mmol, 115 mg) and after that anionic surfactant SDS was added (0.1 mmol, 20 mol% according to (**IIa**)) and neat reaction mixture was intensively stirred (700 rpm) for 20 min at 60 °C. Afterward benzylamine (0.6 mmol, 66 mL) was slowly added (20 min) and such reaction system was stirred at 60 °C for 16 hours. Reaction mixture was diluted with saturated aqueous solution of NaCl (3.5 mL) and gently extracted with EtOAc (35 mL). The organic phase was dried over anhydrous Na₂SO₄ and solvent was evaporated under reduced pressure. The obtained crude product (**Ib**) (105 mg, 61 % yield) was analyzed and confirmed with ¹H NMR spectroscopy.

The reaction was successfully performed (55 % yield) also at ambient temperature at longer reaction time (48 h).

### Example 20: Synthesis of methyl 3-benzylamino-4-(2,4,5-trifluorophenyl)butanoate (Ib) from (IIa) through aza-Michael reaction without adding of solvents in the presence of cetyltrimethylammonium bromide (CTAB) :

In a thick-walled glass vial equipped with an magnetic stir bar was placed methyl (*E*)-4-(2,4,5-trifluorophenyl)-but-2-enoate (**IIa**) (0.5 mmol, 115 mg) and after that cationic surfactant CTAB was added (0.1 mmol, 20 mol% according to (**IIa**)) and neat reaction mixture was intensively stirred (700 rpm) for 20 min at 60 °C. Afterward benzylamine (0.6 mmol, 66 mL) was slowly added (20 min) and such reaction system was stirred at 60 °C for 18 hours. Reaction mixture was diluted with saturated aqueous solution of NaCl (3.5 mL) and gently extracted with EtOAc (35 mL). The organic phase was dried over anhydrous Na₂SO₄ and solvent was evaporated under reduced pressure. The obtained crude product (**Ib**) (98 mg, 57 % yield) was analyzed and confirmed with ¹H NMR spectroscopy.

The reaction was successfully performed (51 % yield) also at ambient temperature at longer reaction time (48 h).

### Example 21: Synthesis of methyl 3-(benzyloxyamino)-4-(2,4,5-trifluorophenyl)butanoate (Ie) from (IIa) in the presence of surfactant-type-Brønsted-acid DBSA in water as reaction medium:

Into a flask equipped with an magnetic stir bar were placed *O*-benzylhydroxylamine hydrochloride (0.7 mmol, 112 mg), NaOH (0.7 mmol, 28 mg) and deonized water was added. After 15 min DBSA (0.1 mmol, 33 mg) was added and reaction system was heated to 60 °C. Afterwards methyl (*E*)-4-(2,4,5-trifluorophenyl)-but-2-enoate (**IIa**) (0.5 mmol, 115 mg) was slowly dropped and such reaction mixture was intensively stirred (900 rpm) at 60 °C for 24 hours. Reaction mixture was diluted with saturated aqueous solution of NaHCO₃ (3.5 mL) and gently extracted with EtOAc (2 x 25 mL). The combined organic phases were finally washed with brine (1 x 40 mL), dried over anhydrous Na₂SO₄ and organic solvent was evaporated under reduced pressure. The obtained crude product (**Ie**) was purified with column chromatography (SiO₂ *n*-hexane : ethylacetate = 2 :1 gradient elution) to obtain (90 mg, 51% yield) of pure product (**Ie**). The product was finally analyzed and confirmed with ¹H, ¹⁹F and ¹³C NMR analysis.
¹H NMR (500 MHz, CDCI₃, ppm) δ 7.35-7.45 (m, 5ArH), 7.10 (m, 1ArH), 6.90 (m, 1ArH), 5.85 (bs, NH), 4.65 (s, 2H), 3.65 (s, 3H), 3.45-3.55 (m, 1H), 2.90 (ddd, *J=* 14 Hz , *J=* 8.7 Hz, *J=* 1.4 Hz, 1H), 2.75 (ddd, *J=* 14 Hz, *J=* 7.8 Hz, *J=* 1.4 Hz, 1H), 2.50 (dd, *J=* 16 Hz, *J=* 7.8 Hz, 1H), 2.45 (dd, *J=* 16 Hz, *J=* 5.2 Hz, 1H).
¹³C (125 MHz, CDCl₃, ppm) δ30.6, 36.0, 51.6, 57.5, 75.2, 105.4 (m), 119.1 (m), 121.5 (m), 127.3, 128.5, 137.5, 145.7 (m, C-F), 149.7 (m, C-F), 157.0 (m, C-F), 172.2 (CO).
¹⁹F NMR (470 MHz, CDCl₃, ppm) δ-119.9 (m), -136.9 (m), -143.9 (m).

### Example 22: Synthesis of methyl 3-(benzyloxyamino)-4-(2,4,5-trifluorophenyl)butanoate (Ie) from (IIa) in water in the presence of 2,2,2-trifluoroethanol (TFE) as promoter:

Into a flask equipped with an magnetic stir bar were placed *O*-benzylhydroxylamine hydrochloride (0.7 mmol, 112 mg), NaOH (0.7 mmol, 28 mg) and deionized water was added. After 15 min TFE (2.5 mmol, 180 mL) was added and such reaction system was heated to 60 °C. Afterwards methyl (*E*)-4-(2,4,5-trifluorophenyl)-but-2-enoate (**IIa**) (0.5 mmol, 115 mg) was slowly added and final reaction mixture was intensively stirred (900 rpm) at 60 °C for 24 hours. Reaction mixture was diluted with saturated aqueous solution of NaCl (3.5 mL) and gently extracted with EtOAc (2 x 25 mL). The combined organic phases were dried over anhydrous Na₂SO₄ and organic solvent was evaporated under reduced pressure. The obtained product (**Ie**) (75 mg, 42 % yield) was analyzed and confirmed with ¹H, ¹⁹F and ¹³C NMR analysis.

### Example 23: Synthesis of methyl 3-(benzyloxy-amino)-4-(2,4,5-trifluorophenyl)butanoate (Ie) from (IIa) in water in the presence of SDS and TFE as promoters:

Into a flask equipped with an magnetic stir bar were placed *O*-benzylhydroxylamine hydrochloride (0.7 mmol, 112 mg), NaOH (0.7 mmol, 28 mg) and deonized water (2.5 mL). Afterward catalytic amount of SDS (0.04 mmol, 12 mg) was added and such reaction system was stirred at ambient temperature for 10 minutes. In the next step methyl (*E*)-4-(2,4,5-trifluorophenyl)-but-2-enoate (**IIa**) was slowly dropped into the reaction mixture and system was heated to 60 °C. Finally the TFE (5 equiv.; 180 µL) was added and such reaction mixture was vigorously stirred (800 rpm) at 60 °C for 15 hours. Reaction mixture was diluted with saturated aqueous solution of NaCl (3.5 mL) and gently extracted with EtOAc (2 x 30 mL). The combined organic phases were dried over anhydrous Na₂SO₄ and organic solvent was evaporated under reduced pressure. The obtained product (**Ie**) (123 mg, 70.5 % yield) was analyzed and confirmed with ¹H, ¹⁹F and ¹³C NMR analysis.

### Example 24: Synthesis of methyl 3-(benzyloxyamino)-4-(2,4,5-trifluorophenyl)butanoate (Ie) from (IIa) in water in the presence of Sc(OTf)₃ and SDS as catalysts:

Into a flask equipped with an magnetic stir bar were placed *O*-benzylhydroxylamine hydrochloride (0.7 mmol, 112 mg), NaOH (0.7 mmol, 28 mg) and deonized water (2.5 mL). Afterward SDS (0.1 mmol; 29 mg; 20 mol% according to substrate) and Sc(OTf)₃ (0.1 mmol; 49 mg; 20 mol%,) and such reaction system was heated to 60 °C. Substrate methyl (*E*)-4-(2,4,5-trifluorophenyl)-but-2-enoate (**IIa**) (0.5 mmol, 115 mg) was slowly added and final reaction mixture was intensively stirred (900 rpm) at 60 °C for 24 hours. Reaction mixture was diluted with saturated aqueous solution of NaCl (3.5 mL) and gently extracted with EtOAc (2 x 25 mL). The combined organic phases were dried over anhydrous Na₂SO₄ and organic solvent was evaporated under reduced pressure. The obtained product (**Ie**) (110 mg, 62 % yield) was analyzed and confirmed with ¹H**,** ¹⁹F and ¹³C NMR analysis.

### Example 25: Synthesis of methyl 3-benzylamino-4-(2,4,5-trifluorophenyl)butanoate (Ib) from (IIa) through aza-Michael reaction in aqueous methanol in the presence of DBSA as catalyst:

In a thick-walled glass vial equipped with an magnetic stir bar was placed methyl (*E*)-4-(2,4,5-trifluorophenyl)-but-2-enoate (**IIa**) (0.5 mmol, 115 mg) which was than vigorously stirred in 2.5 mL of water (900 rpm) to obtain well dispersed aqueous system. Afterwards DBSA (20 mol% according to (**IIa**)) was added, followed by slow addition of methanol (5 mmol, 10 equiv. according to (**IIa**)). The reaction system was heated to 60 °C and benzylamine (0.6 mmol, 66 µL) was slowly added and such reaction mixture was vigorously stirred (900 rpm) at 60 °C for 16 hours. Methanol was first evaporated under reduced pressure, organic aqueous residue was extracted with EtOAc (2 x 35 mL) and combined organic phases were finally washed with brine (1 x 40 mL) and dried over Na₂SO₄. After the solvent was evaporated under reduced pressure, the obtained crude product was purified with column chromatography (SiO₂ *n*-hexane : ethylacetate = 2 : 1) to obtain pure (**Ib**) (98 mg; 58 % yield) which was analyzed and determined with ¹H, ¹³C NMR analysis.

### Example 26: Synthesis of methyl 3-(benzyloxy-amino)-4-(2,4,5-trifluorophenyl)butanoate (Ie) from (IIa) in methanol in the presence of DBSA as catalyst:

Into a flask equipped with an magnetic stir bar were placed *O*-benzylhydroxylamine hydrochloride (0.7 mmol, 112 mg), NaOH (0.7 mmol, 28 mg) and methanol (2.5 mL). During the intensive stiiring of the reaction system, DBSA (20 mol% according to (**IIa**)) was added and such a mixture was heated to 60 °C. Afterward methyl (*E*)-4-(2,4,5-trifluorophenyl)-but-2-enoate (**IIa**) was slowly dropped into the reaction mixture and system was stirred at 60 °C for 20 hours. Methanol was first evaporated, residue was gently extracted with EtOAc (2 x 30 mL). The combined organic phases were dried over anhydrous Na₂SO₄ and organic solvent was evaporated under reduced pressure. The obtained product (le) (85 mg, 48 % yield) was analyzed and confirmed with ¹H and ¹³C NMR analysis.

### Example 27: Synthesis of methyl 3-(phenoxy-amino)-4-(2,4,5-trifluorophenyl)butanoate (Im) from (IIa) in the presence of surfactant-type-Brønsted-acid DBSA in water as reaction medium:

Into a flask equipped with an magnetic stir bar were placed O-phenylhydroxylamine hydrochloride (0.7 mmol, 102 mg), NaOH (0.7 mmol, 28 mg) and deonized water was added. After 15 minutes of stirring, DBSA (0.1 mmol, 33 mg) was added and reaction system was heated to 60 °C under intensive stirring (900 rpm). Afterwards methyl (E)-4-(2,4,5-trifiuorophenyl)-but-2-enoate **(IIa)** (0.5 mmol, 115 mg) was slowly added and such reaction mixture was vigorously stirred at 70 °C for 20 hours. Reaction mixture was diluted with saturated aqueous solution of NaHCO₃ (3.5 mL) and gently extracted with EtOAc (2 x 30 mL). The combined organic phases were finally washed with brine (1 x 40 mL), dried over anhydrous Na₂SO₄ and organic solvent was evaporated under reduced pressure. The obtained product (Im) (68 mg, 40% yield) was analysed and determined with ¹H NMR analysis. ¹H NMR (500 MHz, CDCl₃, ppm) δ 7.45 (m, 2ArH), 7.0-7.35 (m, 3 ArH), 6.80-6.95 (m, 2 ArH), 3.70 (s, 3H), 3.65 (m, 1H), 2.95 (dd, J= 6.8 Hz, J= 14.5 Hz, 1H), 2.80 (dd, J= 7.1 Hz, J= 14.5 Hz, 1H), 2.65 (dd, J= 8.0 Hz, J= 16.5 Hz, 1H), 2.50 (dd, J= 4.9 Hz, J= 16.5 Hz, 1H), 2.35 (bs, NH).

### List of references

WO 03/004498
WO 09/06447
WO 04/085378
WO 05/097733
WO 06/081151
WO 04/085661
WO 04/087650
US 2009/0192326
US 2006/0052382
WO 09/045507
WO 09/045507
WO 2010/122578
Hansen, K. B.; et. al. J. Am. Chem. Soc. 2009, 131, 8798.
Hansen K. B.; et. al. Org. Proc. Res. Dev. 2005, 9, 634.
Hsiao, Y.; et. al. J. Am. Chem. Soc., 2004, 126, 9918.
Kubryl, M.; et. al. Tetrahedron Asymmetry 2006, 17, 205.
Liu, F.; et. al. J. Chem. Res. 4, 2010, 230.
Savile, C. K.; et. al. Science 2010, 329, 305.
Desai, A.; et. al. Angew. Chem. Int. Ed. 2011, 50, 2.
Mutti, F. G.; et. al. ChemCatChem 2011, 3, 109.

The following pages of the description refer to the embodiments of the invention listed as separate items:
1. A process for the preparation of an intermediate of formula I 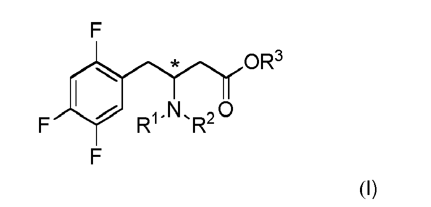 wherein R¹ and R² are identical or different, and are independently selected from
   (i) hydrogen;
   (ii) optionally chiral alkyl residues, having from 1 to 12 carbon atoms, wherein the alkyl residues are optionally aryl and/or aryloxy substituted;
   (iii) optionally chiral alkyloxy residues, having from 1 to 12 carbon atoms, wherein the alkyloxy residues are optionally aryl substituted;
   (iv) optionally chiral aryl residues, having from 6 to 24 carbon atoms, wherein the aryl residues are optionally alkyl and/or alkyloxy substituted;
   (v) optionally chiral aryloxy residues, having from 6 to 24 carbon atoms, wherein the aryloxy residues are optionally alkyl substituted;
   (vi) benzyl;
   (vii) optionally chiral alkaloyl residues, having from 2 to 13 carbon atoms, wherein the alkaloyl residues are optionally aryl substituted;
   (viii) optionally chiral aroyl residues, having from 7 to 25 carbon atoms, wherein the aryloxy residues are optionally alkyl substituted;
   (ix) optionally chiral alkoxycarbonyl residues, having from 2 to 13 carbon atoms;
   (x) optionally chiral aryloxycarbonyl residues, having from 7 to 25 carbon atoms;
   (xi) tosyl;
   (xii) optionally chiral silyl residues, having from 3 to 15 carbon atoms; and
   (xiii) optionally chiral silyloxy residues, having from 3 to 15 carbon atoms;
      wherein R³ is selected from alkyl residues having from 1 to 6 carbon atoms;
      the process comprising the steps of:
      (a) providing an intermediate of formula II, 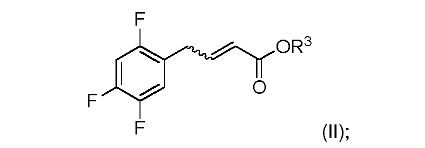
      (b) reacting the intermediate of formula II with an amine of formula III

         HNR¹R² (III)
      wherein R¹ and R² are as defined above, in a protic solvent, particularly in water; or in a mixture of protic solvents, wherein the mixture particularly comprises water; or without adding of solvents in step (b);
      to obtain an intermediate of formula I.
2. The process of item 1, wherein in step (b) the solvent is selected from water, methanol, ethanol, iso-propanol, *tert*-butanol, trifluoroethanol, hexafluoro-2-propanol, amyl alcohol and any combination thereof, and is particularly water.
3. The process of item 1, wherein step (b) is carried out without adding solvents.
4. The process of items 1 to 3, wherein step (b) is carried out at a temperature of 20 °C to 100 °C, preferably of 20 °C to 85 °C.
5. The process of any of items 1 to 4, wherein R¹ and R² are identical, and are selected from
   (i) hydrogen;
   (ii) optionally chiral alkyl residues, having from 1 to 12 carbon atoms, wherein the alkyl residues are optionally aryl and/or aryloxy substituted;
   (iii) optionally chiral aryl residues, having from 6 to 24 carbon atoms, wherein the aryl residues are optionally alkyl and/or alkyloxy substituted;
   (iv) benzyl;
   (v) optionally chiral alkoxycarbonyl residues, having from 2 to 13 carbon atoms;
   (vi) optionally chiral aryloxycarbonyl residues, having from 7 to 25 carbon atoms;
   (vii) optionally chiral silyl residues, having from 3 to 15 carbon atoms;
      and are particularly hydrogen, methyl, *N*-α-methylbenzyl, or trimethylsilyl.
6. The process of any of items 1 to 4, wherein R¹ and R² are different, and are independently selected from
   (i) hydrogen;
   (ii) optionally chiral alkyl residues, having from 1 to 12 carbon atoms, wherein the alkyl residues are optionally aryl and/or aryloxy substituted;
   (iii) optionally chiral alkyloxy residues, having from 1 to 12 carbon atoms, wherein the alkyloxy residues are optionally aryl substituted;
   (iv) optionally chiral aryl residues, having from 6 to 24 carbon atoms, wherein the aryl residues are optionally alkyl and/or alkyloxy substituted;
   (v) optionally chiral aryloxy residues, having from 6 to 24 carbon atoms, wherein the aryloxy residues are optionally alkyl substituted;
   (vi) benzyl;
   (vii) optionally chiral alkaloyl residues, having from 2 to 13 carbon atoms, wherein the alkaloyl residues are optionally aryl substituted;
   (viii) optionally chiral aroyl residues, having from 7 to 25 carbon atoms, wherein the aryloxy residues are optionally alkyl substituted;
   (ix) optionally chiral alkoxycarbonyl residues, having from 2 to 13 carbon atoms;
   (x) optionally chiral aryloxycarbonyl residues, having from 7 to 25 carbon atoms;
   (xi) tosyl;
   (xii) optionally chiral silyl residues, having from 3 to 15 carbon atoms; and
   (xiii) optionally chiral silyloxy residues, having from 3 to 15 carbon atoms;
      and particularly R¹ is hydrogen and R² is tosyl, R¹ is hydrogen and R² is benzyl, R¹ is hydrogen and R² is N-α-methylbenzyl, R¹ is benzyl and R² is *N*-α-methylbenzyl, R¹ is benzyl and R² is N-benzyl-1-phenethyl, R¹ is hydrogen and R² is *O*-benzyl or *O*-methyl, R¹ is hydrogen and R² is *tert*-butyl-oxy-carbonyl or benzyl-oxy-carbonyl, R¹ is hydrogen and R² is methoxy-phenyl, R¹ is hydrogen and R² is *O*-phenyl, or R¹ is hydrogen and R² is O-trimethylsilyl.
7. The process of any of the preceding items, wherein the chiral aryl residues are selected from *N*-α-methylbenzyl, *N*-Bis[α-methylbenzyl], *N*-α-ethyl-naphthyl, 2-methoxybenzyl-1-phenylethyl, 3,4-dimethoxybenzyl-1-phenylethyl, and *N*-benzyl-1-phenethyl.
8. The process of any of the preceding items, wherein R³ is selected from methyl, ethyl, propyl, cyclopropyl, butyl, pentyl, hexyl, isopropyl, isopentyl, and *tert*-butyl, and is particularly methyl.
9. The process of any of items 1 to 4, wherein the intermediate of formula I is 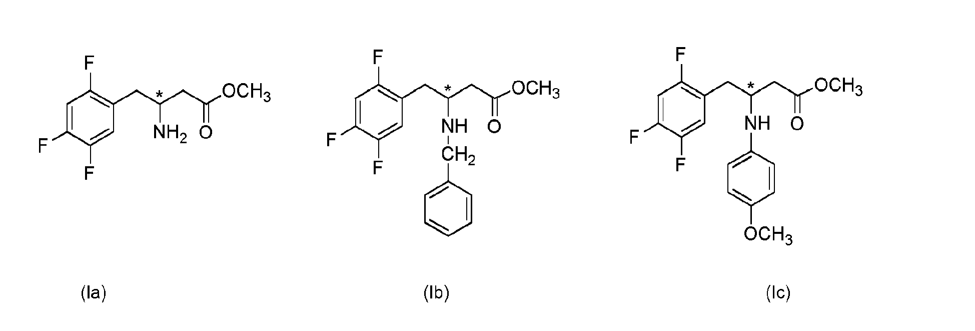 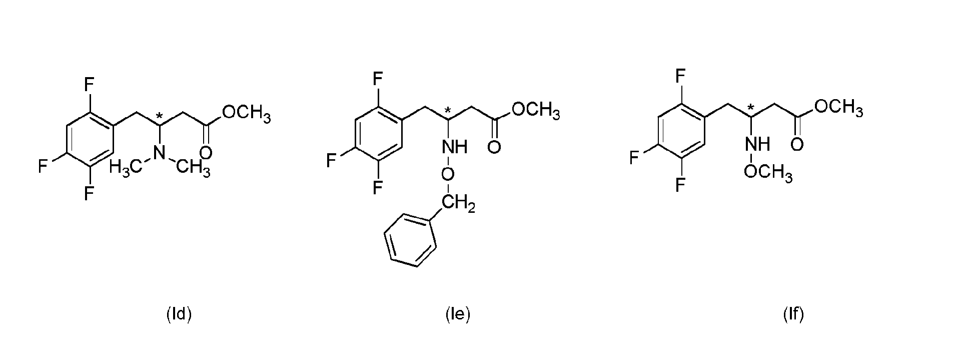 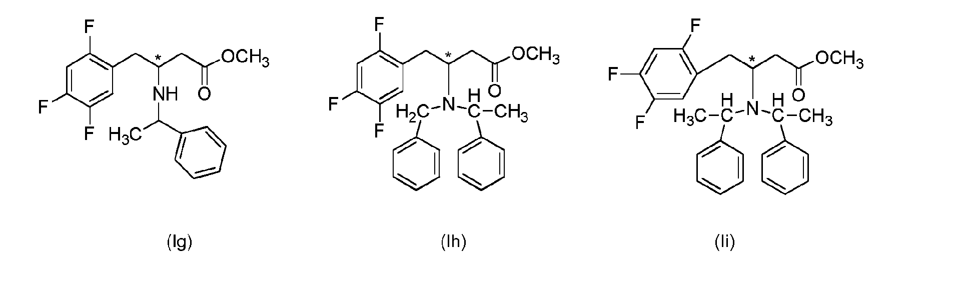 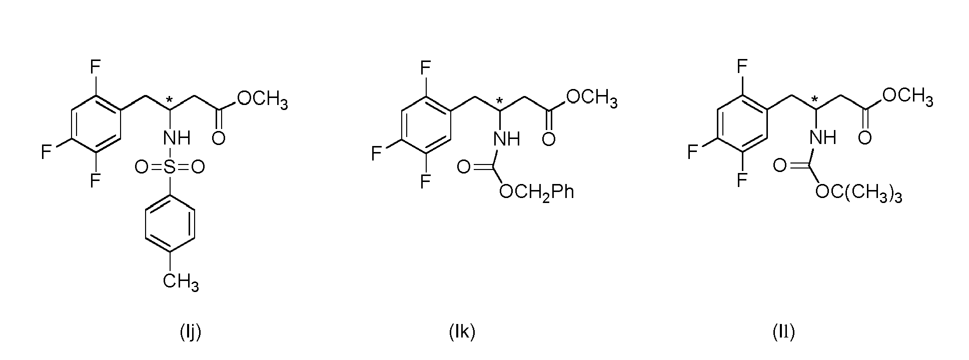 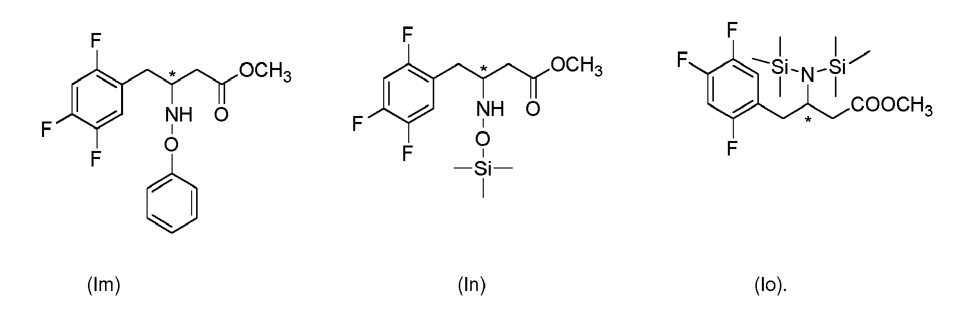
10. The process of any of the preceding items, wherein the amine of formula III is selected from ammonia, an alkyl amine, an aryl amine, an alkyl-aryl amine, a silyl amine, and a silyloxy amine.
11. The process of any of the preceding items, wherein the amine is an alkyl amine and is selected from dimethylamine, *tert*-butyl-carbamate and *O*-methyl-hydroxylamine.
12. The process of any of items 1 to 10, wherein the amine is an aryl amine and is selected from benzylamine, p-methoxybenzylamine, 3,4-dimethoxybenzylamine, *p*-methoxyaniline, tosylamine, benzyl carbamate dibenzylamine, naphythylamine, *O*-benzyl-hydroxylamine, *O*-phenylhydroxylamine and benzhydrylamine.
13. The process of any of items 1 to 10, wherein the amine is an alkyl-aryl amine and is selected from methyl-phenyl-amine, *N*-α-methylbenzylamine, N-bis-[α-methylbenzylamine], and N-benzyl-1-phenylethyl.
14. The process of any of items 1 to 10, wherein the amine is a silyl amine and is selected from hexamethyldisilazane, potassium bis(trimethylsilyl)amide, sodium bis(trimethylsilyl)amide, lithium bis(trimethylsilyl)amide, 1,1,3,3-tetramethyldisilazane and 1,1,3,3-tetramethyl-1,3-diphenylsilazane.
15. The process of any of items 1 to 10, wherein the amine is a silyloxy amine and is selected from *O-*(trimethylsilyl)hydroxylamine, *N*, *O*-bis(trimethylsilyl)hydroxylamine.
16. The process of any of the preceding items, wherein the amine in step (b) is present in an amount of 1.1 to 2.0 equivalents, particularly 1.2 to 1.7 equivalents, with respect to the intermediate of formula II.
17. The process of any of the preceding items, wherein the reaction time is between 5 to 52 hours, particularly between 6 to 48 hours.
18. The process of any of the preceding items, wherein step (b) is a non-catalyzed process.
19. The process of item 18, wherein step (b) is carried out in a protic solvent or in a mixture of protic solvents, and at a temperature of 50 °C to 90 °C, preferably of 60 °C to 85 °C, and more preferably about 60° C.
20. The process of item 18 or 19, wherein step (b) is carried out in a protic solvent or in a mixture of protic solvents, and the reaction time is between 10 to 30 hours, preferably about 16 hours.
21. The process of item 18, wherein step (b) is carried out without adding of solvents, and at a temperature of 25 °C to 90 °C, preferably of 60 °C to 85 °C, and more preferably about 70 °C.
22. The process of item 18 or 21, wherein step (b) is carried out without adding of solvents, and the reaction time is between 8 to 20 hours, preferably about 12 hours.
23. The process of any of items 1 to 17, wherein step (b) is a transition metal catalyzed process, particularly a transition metal catalyzed process using a catalyst comprising a transition metal compound, and optionally at least one ligand.
24. The process of item 23, wherein in step (b) the transition metal compound is selected from copper compounds, indium compounds, zinc compounds, iron compounds, manganese compounds, cerium compounds, bismuth compounds, scandium compounds, ytterbium compounds, yttrium compounds, tin compounds and vanadium compounds, particularly selected from copper compounds, indium compounds, scandium compounds, ytterbium compounds, and iron compounds.
25. The process of item 23 or 24, wherein in step (b) the transition metal compound is a copper compound and is selected from copper(I) acetate, copper(I) chloride, copper(II) chloride, cooper(l) triflate, copper(II) triflate, cooper(II) acetylacetonate, cooper(II) chlorate, and any combination thereof, and is particularly selected from copper(I) acetate, copper(II) triflate, and copper(II) bromide.
26. The process of item 23 or 24, wherein in step (b) the transition metal compound is an indium compound and is selected from indium(III) chloride, indium(II) chloride, indium(III) bromide, indium(III) perchlorate, and indium(III) nitrate, and is particularly indium(III) chloride.
27. The process of item 23 or 24, wherein in step (b) the transition metal compound is a scandium compound and is selected from scandium(III) triflate, scandium(III) perchlorate, scandium(III) chloride, and scandium(III) oxalate, and is particularly scandium(III) triflate.
28. The process of item 23 or 24, wherein in step (b) the transition metal compound is an iron compound and is selected from iron(II) chloride, iron(III) chloride, iron(II) acetylacetonate, iron(III) acetylacetonate, iron(III) chloride hexahydrate, iron(III) triflate, iron(III) chlorate, and iron(III) bromide, and is particularly iron(III) chloride.
29. The process of item 23 or 24, wherein in step (b) the transition metal compound is a vanadium compound and is selected from vanadium(III) acetylacetonate, vanadium(V) oxychloride, vanadium(IV) chloride, and particularly vanadium(IV) chloride or oxychloride, and is particularly vanadium(III) acetylacetonate.
30. The process of any of items 23 to 29, wherein in step (b) the transition metal compound is present in an amount of 2-25 mol%, particularly of 4-20 mol%, and more particularly about 8―15 mol% to the intermediate of formula II.
31. The process of any of items 23 to 30, wherein in step (b) the optionally at least one ligand is selected from monophosphine ligands, diphosphine ligands, and any combination thereof.
32. The process of any of items 23 to 31, wherein in step (b) the optionally at least one ligand is selected from monophosphine ligands, and is particularly triphenylphosphine, tributylphosphine, trimethylphosphine, tricyclohexylphosphine, tri-(*o*-tolyl)-phosphine, tri-(2-furyl)phosphine, tris(dimethylamino)-phosphine, tribenzylphosphine, tripyrolydinophosphine, tris(4-methoxyphenyl)phosphine, and any combination thereof.
33. The process of any of items 23 to 32, wherein in step (b) the optionally at least one ligand is selected from diphosphine ligands, and is particularly 1,2-bis(diphenyl-phosphino)benzene, 1,1,-bis(di-tert-butylphosphino)ferrocene, (oxydi-2,1-phenylene)bis-(diphenylphosphine), (R)-2,2-bis(diphenylphosphino)-1,1-binaphthalene, (*S*)-2,2-bis(diphenylphosphino)-1,1-binaphthale, (*S,R*)-(diphenylphosphino)-ferrocenyl-ethyldi-tert-butylphosphine, (R,S)-(diphenylphosphino)-ferrocenyl-ethyldi-tert-butylphosphine, and any combination thereof.
34. The process of any of items 23 to 33, wherein in step (b) the optionally at least one ligand is present in an amount of 2-20 mol%, particularly of 4―15 mol%, and more particularly 8―10 mol%, with respect to the intermediate of formula II.
35. The process of any of items 23 to 34, wherein the transition metal catalyzed process is optionally carried out in the presence of a base, particularly wherein the base is selected from NaOBut, KOBut, K₂CO₃, Na₂CO₃, KOAc, NaOAc, and any combination thereof, more particularly NaOBut.
36. The process of item 35, wherein the base is present in an amount of 5-25 mol%, particularly of 10-20 mol%, and more particularly about 15 mol%, with respect to the intermediate of formula II.
37. The process of any of items 23 to 34, wherein the transition metal catalyzed process is carried out in the absence of a base.
38. The process of any of items 23 to 37, wherein step (b) is carried out in the presence of a surfactant as defined in any of items 63 to 65.
39. The process of any of items 1 to 17, wherein step (b) is an acid catalyzed process.
40. The process of item 39, wherein in step (b) the acid is a Lewis acid, particularly selected from copper(II) acetate, copper(II) chloride, copper(II) triflate, iron(III) chloride, indium(III) chloride, zinc(II) chloride, scandium(llI) triflate, ytterbium(III) triflate, and vanadium(III) acetylacetonate.
41. The process of item 39, wherein in step (b) the acid is a Brønsted acid, particularly selected from dodecylbenzenesulphonic acid (DBSA), phosphotungstic acid, Nafion-H, trifluoromethanesulphonic acid (HOTf), phosphomolybdic acid, methanesulphonic acid, p-toluenesulphonic acid (PTSA), chlorsulphonic acid 2,5-dinitrobenzenesulphonic acid (DNBSA), sulphuric acid, polystyrenesulfonic acid (PSSA), boric acid, phenyboric acid, and any combination thereof, and is particularly DBSA, PSSA or phosphotungstic acid.
42. The process of any of items 39 to 41, wherein step (b) is carried out in a protic solvent or in a mixture of protic solvents, and in step (b) the acid is present in an amount of 5-30 mol%, particularly of 8-25 mol%, and more particularly about 10-20 mol%, to the intermediate of formula II.
43. The process of any of items 39 to 42, wherein step (b) is carried out in a protic solvent or in a mixture of protic solvents, and step (b) is carried out at a temperature of 25 °C to 90 °C, preferably of 60 °C to 85 °C, and more preferably about 60 °C to 65 °C.
44. The process of any of items 39 to 43, wherein step (b) is carried out in a protic solvent or in a mixture of protic solvents, and the reaction time is between 6 to 24 hours, particularly between 10 to 24 hours.
45. The process of any of items 39 to 41, wherein step (b) is carried out without adding of solvents and in step (b) the acid is present in an amount of 3-30 mol%, particularly of 4-25 mol%, and more particularly about 5-20 mol%, to the intermediate of formula II.
46. The process of any of items 39 to 41 and 45, wherein step (b) is carried out without adding of solvents, and step (b) is carried out at a temperature of 20 °C to 90 °C, preferably of 20 °C to 60 °C.
47. The process of any of items 39 to 41, 45 and 46, wherein step (b) is carried out without adding of solvents, and the reaction time is between 10 to 52 hours, particularly between 12 to 48 hours.
48. The process of any of items 1 to 17, and step (b) is an organocatalyzed process, particularly an organocatalyzed process using an optionally chiral organocatalyst.
49. The process of item 48, wherein in step (b) the organocatalyst is selected from amino acid chiral compounds, particularly selected from pyroglutamic acid, threonine, aspartic acid, and any combination thereof.
50. The process of item 48, wherein in step (b) the organocatalyst is selected from proline derivatives.
51. The process of item 48, wherein in step (b) the organocatalyst is selected from imidazolidinone derivatives.
52. The process of item 48, wherein in step (b) the organocatalyst is selected from cinchona alkaloids.
53. The process of item 48, wherein in step (b) the organocatalyst is selected from; tiourea derivatives.
54. The process of any of items 48 to 53, wherein in step (b) the organocatalyst is present in an amount of 1-30 mol%, particularly of 3-25 mol%, and more particularly 6-20 mol%, to the intermediate of formula II.
55. The process of any of items 48 to 54, wherein step (b) is carried out at a temperature of 25 °C to 90 °C, preferably of 60 °C to 85 °C, and more preferably 60 °C to 80 °C.
56. The process of any of items 48 to 55, wherein the reaction time is between 12 to 24 hours, particularly between 15 to 20 hours.
57. The process of any of items 1 to 17, wherein the step (b) is carried out in the presence of a promoter.
58. The process of item 57, wherein in step (b) the promoter is selected from fluorinated alcohols, in particular selected from trifluoroethanol, hexafluoro-2-propanol, and any combination thereof.
59. The process of item 57 or 58, wherein in step (b) the promoter is present in an amount of 1―15 equivalents, particularly of 3―12 equivalents, and more particularly 5―10 equivalents, to the intermediate of formula II.
60. The process of any of items 57 to 59, wherein step (b) is carried out at a temperature of 50 °C to 90 °C, preferably of 60 °C to 85 °C, and more preferably 60 °C to 80 °C.
61. The process of any of items 57 to 60, wherein the reaction time is between 12 to 30 hours, particularly between 15 to 24 hours.
62. The process of any of the items 1 to 17, wherein step (b) is carried out in the presence of a surfactant.
63. The process of item 62, wherein the surfactant is selected from ionic, nonionic surfactants, and the combination thereof.
64. The process of item 62 or 63, wherein the surfactant is an ionic surfactant and is selected from sodium dodecyl sulfate, sodium stearate, sodium N-lauroylsarcosinate, cetyltrimethylammonium bromide, cetyltrimethylammonium chloride, benzyldodecyammonium bromide, and any combination thereof, and is particularly sodium dodecyl sulfate or cetyltrimethylammonium bromide.
65. The process of item 62 or 63, wherein the surfactant is a nonionic surfactant and is selected from D-α-tocopherol polyethylene glycol succinate, 4-octylphenol polyethoxylate, polyoxyethylene sorbitan monolaurate, polyethylene glycol dodecyl ether, and polyoxyethanyl-α-tocopheryl sebacate, and any combination thereof, and is particularly D-α-tocopherol polyethylene glycol succinate or polyoxyethanyl-α-tocopheryl sebacate.
66. The process of any of items 62 to 65, wherein step (b) is carried out in a protic solvent or in a mixture of protic solvents, and the surfactant is present in an amount of 0.5-30 wt%, particularly of 1-20 wt%, and more particularly 2―15 wt%, with respect to the intermediate of formula II.
67. The process of any of items 62 to 66, wherein step (b) is carried out in a protic solvent or in a mixture of protic solvents, and is carried out at a temperature of 25 °C to 90 °C, preferably of 60 °C to 85 °C, and more preferably 60 °C to 65 °C.
68. The process of any of items 62 to 67, wherein step (b) is carried out in a protic solvent or in a mixture of protic solvents, and the reaction time is between 10 to 20 hours, more particularly between 15 to 20 hours.
69. The process of any of items 62 to 65, wherein step (b) is carried out without adding of solvents, and the surfactant is present in an amount of 5―40 wt%, particularly of 10-30 wt%, and more particularly 15-20 wt%, with respect to the intermediate of formula II.
70. The process of any of items 62 to 65 and 69, wherein step (b) is carried out without adding of solvents, and is carried out at a temperature of 25 °C to 90 °C, preferably of 60 °C to 85 °C, and more preferably 60 °C to 65 °C.
71. The process of any of items 62 to 65, 69 and 70, wherein step (b) is carried out without adding of solvents, and the reaction time is between 10 to 20 hours, more particularly between 16 to 18 hours.
72. The process of item 1, wherein R¹ is hydrogen, R² is benzyl and R³ is methyl;
   the process comprises or consists the steps of:
   (a) providing an intermediate of formula II, wherein R³ is methyl; 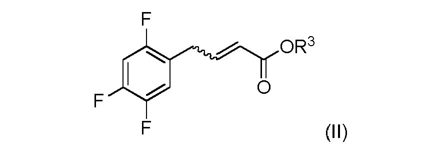
   (b) reacting the intermediate of formula II with benzylamine present in an amount of 1.1 to 1.5 equivalents, preferably about 1.2 equivalents, with respect to the intermediate of formula II, in water at 20 °C to 65°C, preferably at 60 °C, for 10 to 30 hours, preferably 24 hours, to obtain an intermediate of formula I.
73. The process of item 1, wherein R¹ is hydrogen, R² is benzyl and R³ is methyl;
   the process comprises or consists the steps of:
   (a) providing an intermediate of formula II, wherein R³ is methyl; 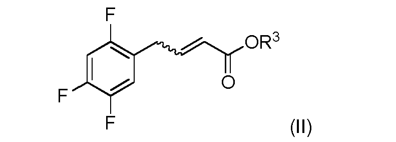
   (b) reacting the intermediate of formula II with benzylamine present in an amount of 1.1 to 1.5 equivalents, preferably about 1.2 equivalents, with respect to the intermediate of formula II, in water at 30 °C for 5 to 7 hours, preferably 6 hours,
   in the presence of
   (i) copper(II) acetate, preferably present in an amount of 4-12 mol%, more preferably about 10 mol%, with respect to the intermediate of formula II;
   (ii) triphenylphosphine, preferably present in an amount of 4―12 mol%, more preferably 8―10 mol%, with respect to the intermediate of formula II,
   (iii) sodium dodecylsulfate, preferably present in an amount of 4-20 mol%, more preferably 8―10 mol%, with respect to the intermediate of formula II, and
   (iv) NaOBut, preferably present in an amount of 10-20 mol%, more preferably about 15 mol%, with respect to the intermediate of formula II,
   to obtain an intermediate of formula I.
74. The process of item 1, wherein R¹ is hydrogen, R² is benzyl and R³ is methyl;
   the process comprises or consists the steps of:
   (a) providing an intermediate of formula II, wherein R³ is methyl; 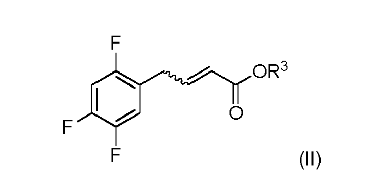
   (b) reacting the intermediate of formula II with benzylamine present in an amount of 1.1 to 1.5 equivalents, preferably about 1.2 equivalents, with respect to the intermediate of formula II, in water at 30 °C to 65 °C, preferably at 60 °C, for 5 to 7 hours, preferably 6 hours,
      in the presence of
      (i) copper(II) acetate, preferably present in an amount of 4-12 mol%, more preferably about 10 mol%, with respect to the intermediate of formula II;
      (ii) sodium dodecylsulfate, preferably present in an amount of 4-20 mol%, more preferably 8―10 mol%, with respect to the intermediate of formula II, and
      to obtain an intermediate of formula I.
75. The process of item 1, wherein R¹ is hydrogen, R² is benzyl and R³ is methyl;
   the process comprises or consists the steps of:
   (a) providing an intermediate of formula II, wherein R³ is methyl; 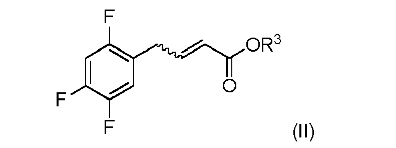
   (b) reacting the intermediate of formula II with benzylamine present in an amount of 1.1 to 1.5 equivalents, preferably about 1.2 equivalents, with respect to the intermediate of formula II, in water at 50 °C to 95 °C, preferably at 80 °C, for 10 to 20 hours, preferably 15 hours,
      in the presence of
      (i) copper(II) bromide, preferably present in an amount of 10-20 mol%, more preferably about 15 mol%, with respect to the intermediate of formula II; or
      (ii) iron(III) chloride, preferably present in an amount of 10-20 mol%, more preferably about 15 mol%, with respect to the intermediate of formula II;
      to obtain an intermediate of formula I.
76. The process of item 1, wherein R¹ is hydrogen, R² is benzyl and R³ is methyl;
   the process comprises or consists the steps of:
   (a) providing an intermediate of formula II, wherein R³ is methyl; 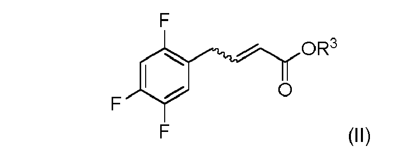
   (b) reacting the intermediate of formula II with benzylamine present in an amount of 1.1 to 1.5 equivalents, preferably about 1.2 equivalents, with respect to the intermediate of formula II, in water at 25 °C to 90 °C, preferably at 60 °C, for 15 to 25 hours, preferably 20 hours,
      in the presence of
      (i) indium(III) chloride, preferably present in an amount of 10-20 mol%, more preferably about 15 mol%, with respect to the intermediate of formula II;
      to obtain an intermediate of formula I.
77. The process of item 1, wherein R¹ is hydrogen, R² is benzyl and R³ is methyl;
   the process comprises or consists the steps of:
   (a) providing an intermediate of formula II, wherein R³ is methyl; 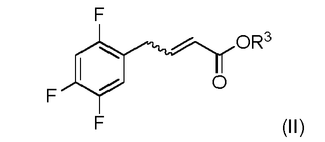
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 80 °C, for 10 to 20 hours, preferably 16 hours,
      in the presence of
      (i) 2,2,2-trifluoroethanol, preferably present in an amount of 5―15 equivalents, more preferably about 5-10 equivalents, with respect to the intermediate of formula II;
      to obtain an intermediate of formula I.
78. The process of item 1, wherein R¹ is hydrogen, R² is benzyl and R³ is methyl;
   the process comprises or consists the steps of:
   (a) providing an intermediate of formula II, wherein R³ is methyl; 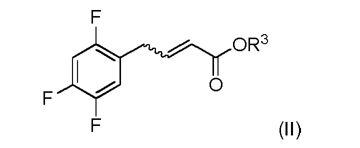
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 15 to 25 hours, preferably 20 hours,
      in the presence of
      (i) 1,1,1,3,3,3-hexafluoro-2-propanol, preferably present in an amount of 5-15 equivalents, more preferably about 5-10 equivalents, with respect to the intermediate of formula II;
      to obtain an intermediate of formula I.
79. The process of item 1, wherein R¹ is hydrogen, R² is benzyl and R³ is methyl;
   the process comprises or consists the steps of:
   (a) providing an intermediate of formula II, wherein R³ is methyl; 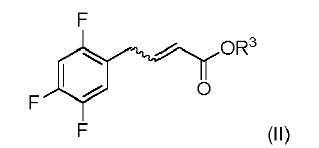
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 20 hours, preferably 15 hours,
      in the presence of
      (i) D-α-tocopherol-polyethyleneglycol-succinate, preferably present in an amount of 2-15 wt% with respect to the intermediate of formula II; or
      (ii) polyoxyethanyl-α-tocopheryl-sebacate, preferably present in an amount of 2―15 wt% with respect to the intermediate of formula II;
      to obtain an intermediate of formula I.
80. The process of item 1, wherein R¹ is hydrogen, R² is benzyl and R³ is methyl;
   the process comprises or consists the steps of:
   (a) providing an intermediate of formula II, wherein R³ is methyl; 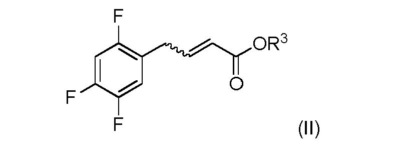
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 60 °C, for 15 to 25 hours, preferably 20 hours,
      in the presence of
      (i) sodium dodecylsulfate, preferably present in an amount of 2―15 wt% with respect to the intermediate of formula II;
      to obtain an intermediate of formula I.
81. The process of item 1, wherein R¹ is hydrogen, R² is benzyl and R³ is methyl;
   the process comprises or consists the steps of:
   (a) providing an intermediate of formula II, wherein R³ is methyl; 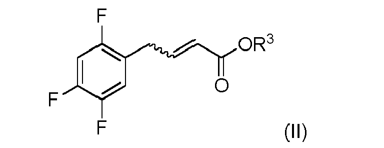
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 5 to 20 hours, preferably 12 hours,
      in the presence of
      (i) 4-dodecybenzenesulfonic acid (DBSA), preferably present in an amount of 15-25 mol%, more preferably about 20 mol%, with respect to the intermediate of formula II;
      to obtain an intermediate of formula I.
82. The process of item 1, wherein R¹ is hydrogen, R² is benzyl and R³ is methyl;
   the process comprises or consists the steps of:
   (a) providing an intermediate of formula II, wherein R³ is methyl; 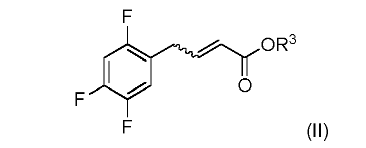
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 15 hours, preferably 20 hours,
      in the presence of
      (i) polystyrenesulfonic acid (PSSA), preferably present in an amount of 5-15 mol%, more preferably about 10 mol%, with respect to the intermediate of formula II;
      to obtain an intermediate of formula I.
83. The process of item 1, wherein R¹ is hydrogen, R² is benzyl and R³ is methyl;
   the process comprises or consists the steps of:
   (a) providing an intermediate of formula II, wherein R³ is methyl; 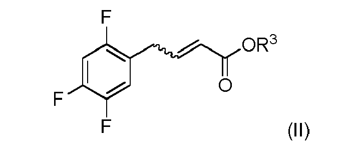
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 25 hours, preferably 15 hours,
      in the presence of
      (i) phosphotungstic acid, preferably present in an amount of 5―15 mol%, more preferably about 10 mol%, with respect to the intermediate of formula II;
      to obtain an intermediate of formula I.
84. The process of item 1, wherein R¹ is hydrogen, R² is benzyl and R³ is methyl;
   the process comprises or consists the steps of:
   (a) providing an intermediate of formula II, wherein R³ is methyl; 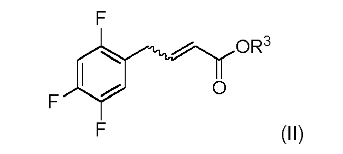
   (b) reacting the intermediate of formula II with benzylamine present in an amount of 1.1 to 1.5 equivalents, preferably about 1.2 equivalents, with respect to the intermediate of formula II, in water at 50 °C to 90 °C, preferably at 65 °C, for 10 to 25 hours, preferably 18 hours,
      in the presence of
      (i) acid activator Nafion NR50, preferably present in an amount of 5―15 mol%, more preferably about 10 mol%, with respect to the intermediate of formula II;
      to obtain an intermediate of formula I.
85. The process of item 1, wherein R¹ is hydrogen, R² is benzyl and R³ is methyl;
   the process comprises or consists the steps of:
   (a) providing an intermediate of formula II, wherein R³ is methyl; 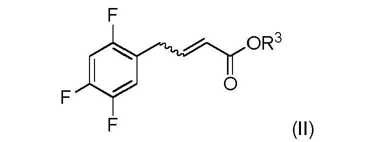
   (b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 70 °C, for 8 to 20 hours, preferably 12 hours, to obtain an intermediate of formula I.
86. The process of item 1, wherein R¹ is hydrogen, R² is benzyl and R³ is methyl;
   the process comprises or consists the steps of:
   (a) providing an intermediate of formula II, wherein R³ is methyl; 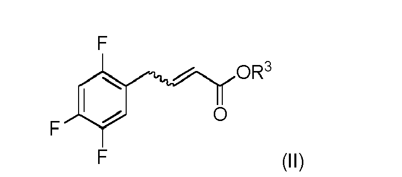
   (b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 50 °C, preferably at ambient temperature, for 24 to 52 hours, preferably 48 hours,
      in the presence of
      (i) 4-dodecybenzenesulfonic acid (DBSA), preferably present in an amount of 15-25 mol%, more preferably about 20 mol%, with respect to the intermediate of formula II;
      to obtain an intermediate of formula I.
87. The process of item 1, wherein R¹ is hydrogen, R² is benzyl and R³ is methyl;
   the process comprises or consists the steps of:
   (a) providing an intermediate of formula II, wherein R³ is methyl; 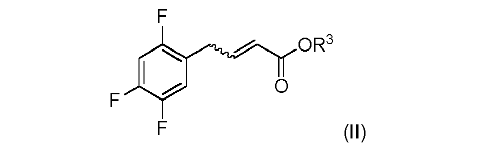
   (b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 60 °C, for 10 to 25 hours, preferably 18 hours,
      in the presence of
      (i) phosphotungstic acid, preferably present in an amount of 3―10 mol%, more preferably about 5 mol%, with respect to the intermediate of formula II;
      to obtain an intermediate of formula I.
88. The process of item 1, wherein R¹ is hydrogen, R² is benzyl and R³ is methyl;
   the process comprises or consists the steps of:
   (a) providing an intermediate of formula II, wherein R³ is methyl; 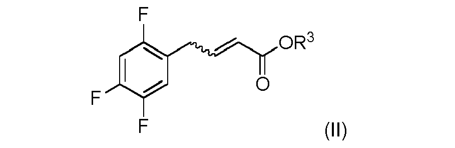
   (b) reacting the intermediate of formula II with benzylamine present in an amount of 1.1 to 1.5 equivalents, preferably about 1.2 equivalents, with respect to the intermediate of formula II, without adding of solvents in step (a) and step (b) at 25 °C to 90 °C, preferably at 60 °C, for 10 to 25 hours,
      preferably 16 hours,
      in the presence of
      (i) sodium dodecylsulfate, preferably present in an amount of 10-30 mol%, more preferably about 20 mol%, with respect to the intermediate of formula II,
      to obtain an intermediate of formula I.
89. The process of item 1, wherein R¹ is hydrogen, R² is benzyl and R³ is methyl;
   the process comprises or consists the steps of:
   (a) providing an intermediate of formula II, wherein R³ is methyl; 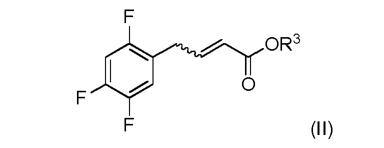
   (b) reacting the intermediate of formula II with benzylamine present in an amount of 1.1 to 1.5 equivalents, preferably about 1.2 equivalents, with respect to the intermediate of formula II, without adding of solvents in the step (b) at 25 °C to 90 °C, preferably at 60 °C, for 10 to 25 hours, preferably 18 hours,
      in the presence of
      (i) cetyltrimethylammonium bromide, preferably present in an amount of 10-30 mol%, more preferably about 20 mol%, with respect to the intermediate of formula II,
      to obtain an intermediate of formula I.
90. The process of item 1, wherein R¹ is hydrogen, R² is O-benzyl and R³ is methyl;
   the process comprises or consists the steps of:
   (a) providing an intermediate of formula II, wherein R³ is methyl; 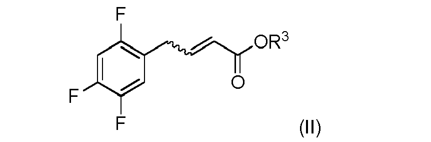
   (b) reacting the intermediate of formula II with O-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
      in the presence of
      (i) 4-dodecybenzenesulfonic acid (DBSA), preferably present in an amount of 15-25 mol%, more preferably about 20 mol%, with respect to the intermediate of formula II;
      to obtain an intermediate of formula I.
91. The process of item 1, wherein R¹ is hydrogen, R² is *O*-benzyl and R³ is methyl;
   the process comprises or consists the steps of:
   (a) providing an intermediate of formula II, wherein R³ is methyl; 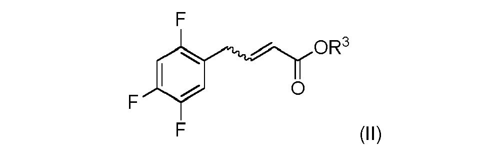
   (b) reacting the intermediate of formula II with O-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
      in the presence of
      (i) 2,2,2-trifluoroethanol, preferably present in an amount of 1―15 equivalents, preferably about 3-12 equivalents, more preferably about 5 equivalents with respect to the intermediate of formula II;
      to obtain an intermediate of formula I.
92. The process of item 1, wherein R¹ is hydrogen, R² is O-benzyl and R³ is methyl; and the process comprises or consists the steps of:
   (a) providing an intermediate of formula II, wherein R³ is methyl; 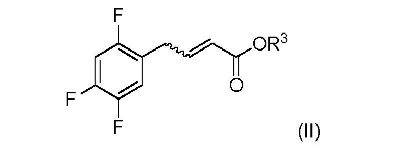
   (b) reacting the intermediate of formula II with O-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
      in the presence of
      (i) scandium(III) triflate, preferably present in an amount of 1-30 mo%, preferably about 5-25 mol%, more preferably about 20 mol%, with respect to the intermediate of formula II; and
      (ii) sodium dodecylsulfate, present in an amount of 1-30 mol%, preferably about 5-25 mol%, more preferably about 20 mol%, with respect to the intermediate of II;
      to obtain an intermediate of formula I.
93. The process of item 1, wherein R¹ is hydrogen, R² is O-benzyl and R³ is methyl; and
   the process comprises or consists the steps of:
   (a) providing an intermediate of formula II, wherein R³ is methyl; 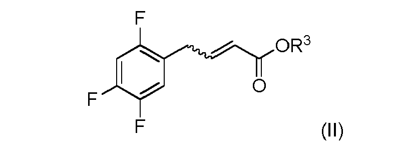
   (b) reacting the intermediate of formula II with O-benzylhydroxylamine present in an amount of 1.1 to 2.0 equivalents, preferably about 1.4 equivalents, with respect to the intermediate of formula II, in water at 50 °C to 90 °C, preferably at 60 °C, for 10 to 30 hours, preferably 24 hours,
      in the presence of
      (i) 2,2,2-trifluoroethanol, present in an amount of 1-15 equivalents, preferably about 3-12 equivalents, more preferably about 5 equivalents, with respect to the intermediate of formula II; and
      (ii) sodium dodecylsulfate, preferably present in an amount of 1-30 mol%, preferably about 5-20 mol%, more preferably about 8 mol%, with respect to the intermediate of II;
      to obtain an intermediate of formula I.
94. Use of a process of any of preceding items in a process for the preparation of anti-diabetic agents, in particular *(R)*-3-Amino-1-[3-(trifluormethyl)-5,6,7,8-tetrahydro[1,2,4]triazol[4,3-a]pyrazin-7-yl]-4-(2,4,5-trifluorphenyl)butan-1-on.

## Claims

1. A process for the preparation of an intermediate of formula I 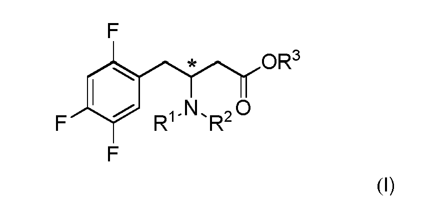 wherein R¹ and R² are identical or different, and are independently selected from
(i) hydrogen;
(ii) optionally chiral alkyl residues, having from 1 to 12 carbon atoms, wherein the alkyl residues are optionally aryl and/or aryloxy substituted;
(iii) optionally chiral alkyloxy residues, having from 1 to 12 carbon atoms, wherein the alkyloxy residues are optionally aryl substituted;
(iv) optionally chiral aryl residues, having from 6 to 24 carbon atoms, wherein the aryl residues are optionally alkyl and/or alkyloxy substituted;
(v) optionally chiral aryloxy residues, having from 6 to 24 carbon atoms, wherein the aryloxy residues are optionally alkyl substituted;
(vi) benzyl;
(vii) optionally chiral alkaloyl residues, having from 2 to 13 carbon atoms, wherein the alkaloyl residues are optionally aryl substituted;
(viii) optionally chiral aroyl residues, having from 7 to 25 carbon atoms, wherein the aryloxy residues are optionally alkyl substituted; and
(ix) optionally chiral alkoxycarbonyl residues, having from 2 to 13 carbon atoms;
(x) optionally chiral aryloxycarbonyl residues, having from 7 to 25 carbon atoms;
(xi) tosyl;
(xii) optionally chiral silyl residues, having from 3 to 15 carbon atoms; and
(xiii) optionally chiral silyloxy residues, having from 3 to 15 carbon atoms;
wherein R³ is selected from alkyl residues having from 1 to 6 carbon atoms;
the process comprising the steps of:
(a) providing an intermediate of formula II, 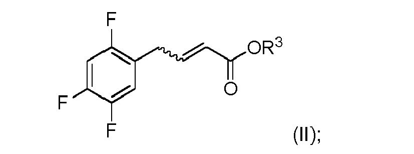
(b) reacting the intermediate of formula II with an amine of formula III
HNR¹R² (III)
wherein R¹ and R² are as defined above, in a protic solvent, particularly in water; or in a mixture of protic solvents, wherein the mixture particularly comprises water; or without adding of solvents in step (b);
to obtain an intermediate of formula I.

2. The process of claim 1, wherein in step (b) the solvent is selected from water, methanol, ethanol, iso-propanol, tert-butanol, trifluoroethanol, hexafluoro-2-propanol, amyl alcohol and any combination thereof, and is particularly water.

3. The process of claim 1, wherein step (b) is carried out without adding solvents.

4. The process of any of the preceding claims, wherein step (b) is carried out at a temperature of 20 °C to 100 °C, preferably of 20 °C to 85 °C.

5. The process of any of the preceding claims, wherein the intermediate of formula I is

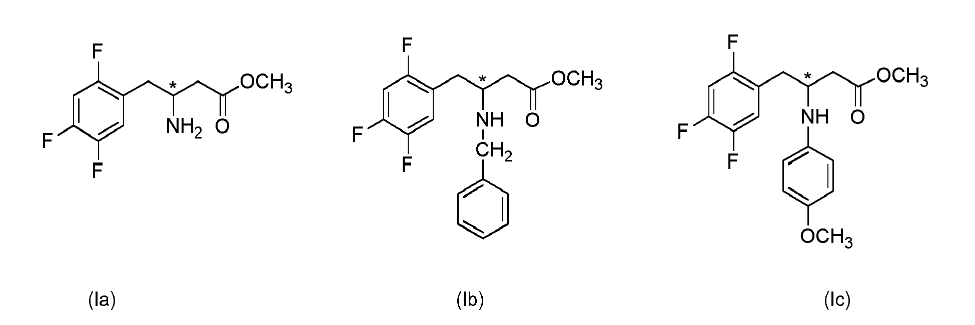

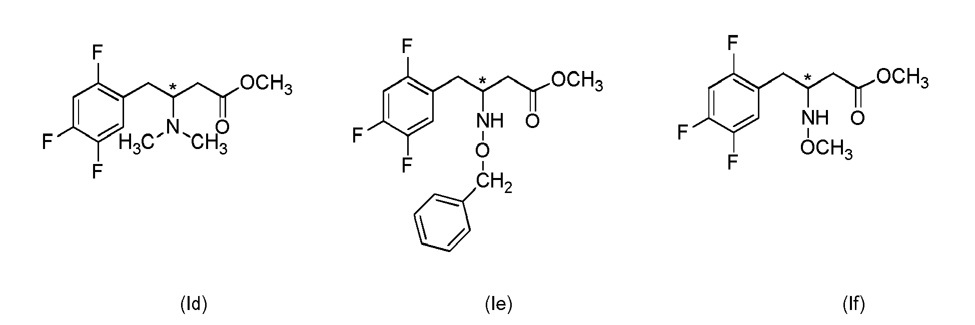

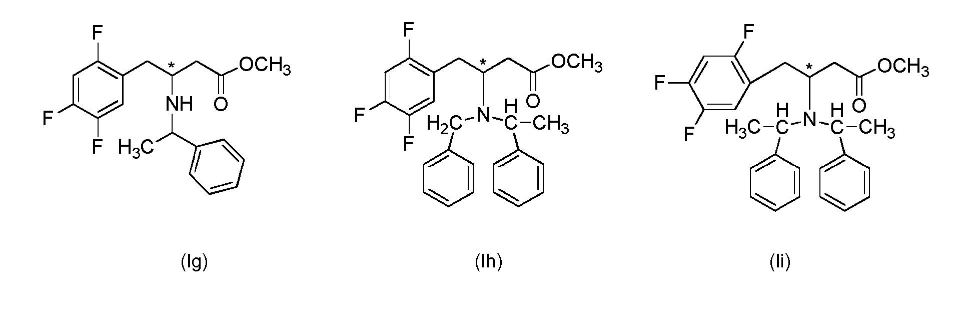

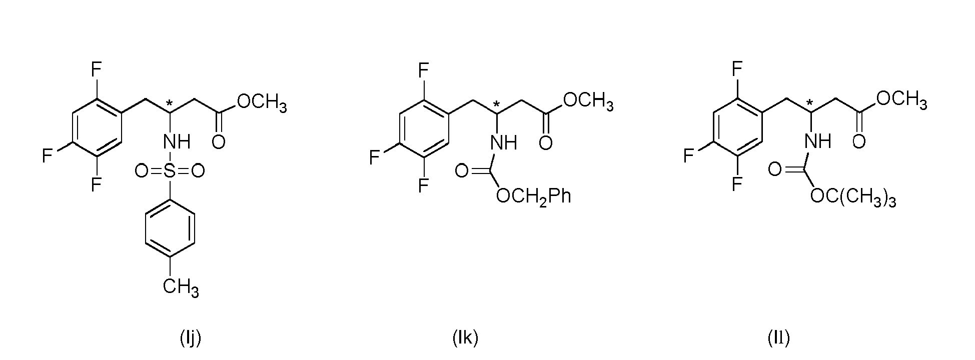

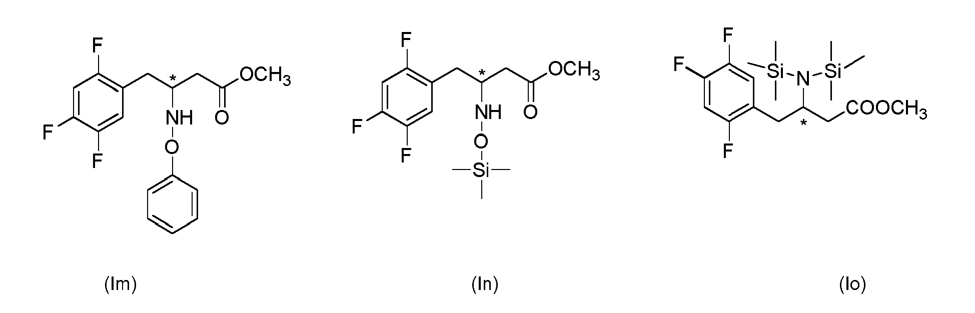

6. The process of any of the preceding claims, wherein step (b) is a non-catalyzed process.

7. The process of any of claims 1 to 5 wherein step (b) is a transition metal catalyzed process, particularly a transition metal catalyzed process using a catalyst comprising a transition metal compound, and optionally at least one ligand.

8. The process of claim 7, wherein in step (b) the transition metal compound is selected from copper compounds, indium compounds, zinc compounds, iron compounds, manganese compounds, cerium compounds, bismuth compounds, scandium compounds, ytterbium compounds, yttrium compounds, tin compounds and vanadium compounds, particularly selected from copper compounds, indium compounds, scandium compounds, ytterbium compounds, and iron compounds.

9. The process of claim 7 or 8, wherein the transition metal catalyzed process is optionally carried out in the presence of a base, particularly wherein the base is selected from NaOBut, KOBut, K₂CO₃, Na₂CO₃, KOAc, NaOAc, and any combination thereof, more particularly NaOBut.

10. The process of claim 7 or 8, wherein the transition metal catalyzed process is carried out in the absence of a base.

11. The process of any of claims 1 to 5, wherein step (b) is an acid catalyzed process.

12. The process of claim 11, wherein in step (b) the acid is a Lewis acid, particularly selected from copper(II) acetate, copper(II) chloride, copper(II) triflate, iron(III) chloride, indium(III) chloride, scandium(III) triflate, ytterbium(III) triflate, and vanadium(III) acetyacetonate.

13. The process of claim 11, wherein in step (b) the acid is a Brønsted acid, particularly selected from dodecylbenzenesulphonic acid (DBSA), phosphotungstic acid, Nafion-H, trifluoromethanesulphonic acid (HOTf), phosphomolybdic acid, methanesulphonic acid, p-toluenesulphonic acid (PTSA), chlorsulphonic acid 2,5-dinitrobenzenesulphonic acid (DNBSA), sulphuric acid, polystyrenesulfonic acid (PSSA), boric acid, phenyboric acid, and any combination thereof, and is particularly DBSA, PSSA or phosphotungstic acid.

14. The process of any of claims 1 to 5, wherein step (b) is an organocatalyzed process, particularly an organocatalyzed process using an optionally chiral organocatalyst.

15. The process of any of claims 1 to 5, wherein step (b) is carried out in the presence of a promoter, in particular selected from fluorinated alcohols, in particular selected from trifluoroethanol, hexafluoro-2-propanol, and any combination thereof.

16. The process of any of the claims 1 to 5, wherein step (b) is carried out in the presence of a surfactant, in particular wherein the surfactant is selected from ionic, nonionic surfactants, and the combination thereof.

17. Use of a process of any of claims 1 to 16 in a process for the preparation of anti-diabetic agents, in particular (R)-3-Amino-1-[3-(trifluormethyl)-5,6,7,8-tetrahydro[1,2,4]triazol[4,3-a]pyrazin-7-yl]-4-(2,4,5-trifluorphenyl)butan-1-on.
